(19) Europäisches Patentamt
European Patent Office
Office européen des brevets

(11) EP 2 944 647 A1

(12) **EUROPEAN PATENT APPLICATION**

(43) Date of publication:
**18.11.2015 Bulletin 2015/47**

(51) Int Cl.:
***C07H 21/00*** *(2006.01)* ***C12Q 1/68*** *(2006.01)*

(21) Application number: **15167287.0**

(22) Date of filing: **25.07.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.07.2007 US 962258 P**

(62) Document number(s) of the earlier application(s) in accordance with Art. 76 EPC:
**08796660.2 / 2 185 573**

(71) Applicants:
- **Cellay, Inc.**
  **Cambridge, Massachusetts 02139 (US)**
- **Moen, Phillip T. Jr.**
  **Cambridge, MA 02139 (US)**

(72) Inventors:
- **Moen Jr., Phillip T.**
  **Cambridge, MA 02139 (US)**
- **Aurich-Costa, Joan**
  **Cambridge, MA 02139 (US)**
- **Bradley, Sean P.**
  **Cambridge, MA 02139 (US)**

(74) Representative: **Potter Clarkson LLP**
**The Belgrave Centre**
**Talbot Street**
**Nottingham NG1 5GG (GB)**

Remarks:
This application was filed on 12-05-2015 as a divisional application to the application mentioned under INID code 62.

(54) **HIGHLY VISIBLE CHROMOSOME-SPECIFIC PROBES AND RELATED METHODS**

(57) Disclosed are chromosome-specific synthetic oligonucleotides and labelled probe compositions, as well as methods for preparing and using such compositions. Also disclosed are kits for utilisation in methods for preparing or using the labelled probes. The probes comprise chromosome-specific sequences consisting of 25-35 contiguous nucleotides perfectly complementary to a repeat sequence on a specific human chromosome, but less than 84% identical to a consensus repeat sequence of all human chromosomes. The probes may comprise a hairpin structure to which a plurality of labels are attached.

Figure 1

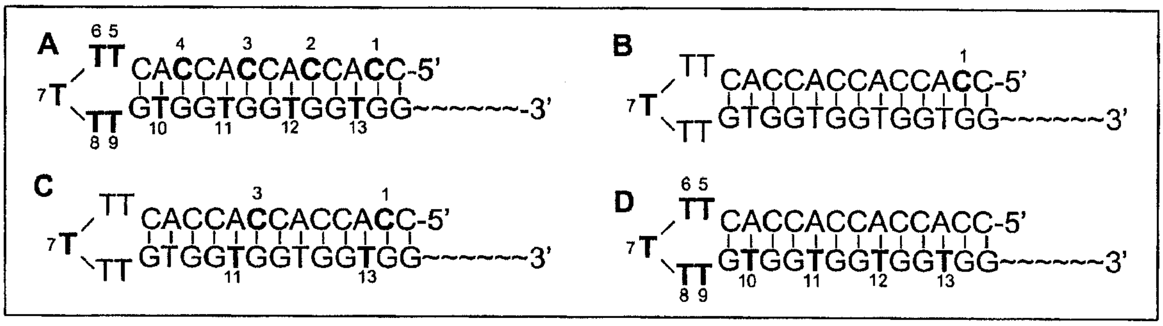




Printed by Jouve, 75001 PARIS (FR)

## Description

### CROSS-REFERENCE TO RELATED APPLICATIONS

This application claims the benefit under 35 U.S.C. 119(e) of U.S. Provisional Patent Application No. 60/962,258, filed July 26, 2007, which is incorporated herein by reference in its entirety for all purposes.

### REFERENCE TO A SEQUENCE LISTING

This application includes a sequence listing in a text file entitled SEQLIS019168-000910PC.txt, created on July 25, 2008, and containing 3666 bytes. The material contained in the text file is hereby incorporated by reference. The sequence listing information recorded in computer readable form is identical to the written sequence listing appended hereto.

### BACKGROUND OF THE INVENTION

Detection of specific chromosomes is important to a variety of diagnostic methods. For example, one nucleic acid hybridization technique commonly used for detection of target chromosomes is Fluorescent *In Situ* Hybridization (FISH). The high degree of specificity and accuracy, and the ability to generate rapid results, have made FISH the method of choice for identifying, *e.g.,* chromosomal abnormalities, genetic diseases, and cancers. *(See* Heim and Mitelman, Cancer Cytogenetics, Chromosomal and Molecular Genetic Aberrations of Tumor Cells, 1995; Klinger, Cytogenetics Analysis, Non-Isotopic Methods in Molecular Biology, A Practical Approach, 1995; Timm et al., Cytometry 22:250-5, 1995; Heselmeyer et al., Genes, Chromosomes & Cancer 19:233-40, 1997; Sauer et al., Apmis 111:444-50, 2003). In prenatal diagnosis, FISH is commonly used for detecting aneuploidies. It has recently been demonstrated that 83% of all chromosome abnormalities present in spontaneous abortions can be detected by FISH *(See* Jobanputra et al., Hum. Reprod. 17:1166-70, 2002.) A series of recent publications has highlighted FISH as an important analytical tool in the relatively new field of Preimplantation Genetic Diagnosis (PGD). *(See, e.g.,* Munné et al., Reprod Biomed Online 7:91-7, 2003.) The increasing use of techniques for reproductive assistance methods, such as *in vitro* fertilization (IVF), have made it necessary to develop new methods for determining which fertilized eggs are the best candidates for implantation. Surprisingly, data has shown that despite appearing morphologically normal, the majority of embryos derived from IVF exhibit varying degrees of chromosomal aneuploidy. *(See* Marquez et al., Reprod Biomed Online 1:729-31, 2000; Abdelhadi et al., Reprod Biomed Online 6:226-31, 2003.) Furthermore, the use of interphase FISH prior to IVF has doubled the rate of successful implantations. *(See* Gianaroli et al., Fertil Steril 72:837-44, 1999.)

Over 100 years ago, von Hansemann (Von Hansemann D., "Ueber asymmetrische Zelltheilung in epithelkrebsen und deren biologische bedeutung," Virchow's Arch Pathol Anat 119:299-326 (1890)) first described the observation of aneuploidy in malignant tumors. More recently it has been suggested that the degree of aneuploidy in cells reflects well the cell's proclivity for genomic instability *(*Duesberg et al., "Genetic instability of cancer cells is proportional to their degree of aneuploidy," Proc Natl Acad Sci USA 95:13692-13697 (1998)). Early in the $20^{th}$ century, Theodore Boveri suggested that aneuploidy was responsible for cancer, but interest in his theory was lost after his death in 1915 *(*Duesberg et al., "Aneuploidy the somatic mutation that makes cancer a species on its own," Cell Motil. Cytoskeleton 47:81-107 (2000)). Aneuploidy had been since then considered as a consequence rather than a cause of genetic instability in malignant transformation. As further insights into genetics of solid tumors have unfolded, the aneuploidy hypothesis has been rekindled since the most common aberration found in over 20,000 solid tumors is chromosome number alterations (Bialy H., "Aneuploidy and cancer: vintage wine in a new bottle?," Nat Biotechnol., 16(2):137-8 (1998); Sen S., "Aneuploidy and cancer," Curr Opin Oncol., 12(1):82-8 (2000)). Aneuploidy has been described in many human tumors, and can serve as a marker of malignant neoplasms. In fact, the FDA has approved a detection kit for bladder cancer for clinical use in aneuploidy detection by FISH using the most commonly altered chromosomes 3, 7, 9 and 17. Sokolova et al., J Mol Diagn. 2(3):116-23 (2000). The FISH probes currently used in such detection methods are derived from genomic DNA fragments cloned into plasmids or BACs and require an overnight hybridization to obtain optimum results.

The presence of defined centromeric repeat sequences permits the use of smaller and more precise synthetic oligodeoxynucleotide probes (ODN probes), instead of the more commonly used probes derived from large genomic DNA sequences (Matera et al., Genomics 18:729-31, 1993). ODNs generally hybridize more rapidly, are more consistent, and are cheaper to manufacture. Some current chromosome-specific oligonucleotides are described in, *e.g.,* U.S. Patent No. 6,300,066; Pellestor et al. (Cytogenet. Cell Genet. 70:138-142 (1995); Anston et al. (Eur. J. Hum. Genet. 11:357-363, 2003); Paulasova et al. (Mol. Hum. Reprod. 10:467-472, 2004); and O'Keefe and Matera (Genome Res. 10:1342-1350, 2000).

Smaller ODN probes designed to target and/or detect repeat sequences, however, have typically lacked sufficient detection sensitivity and/or specificity. For example, detection using conventional chromosome-specific oligonucleotide probes typically requires sets of indirectly labeled specific probes and/or signal amplification, digital enhancement or integration in order to detect a signal. Accordingly, there is a need in the art for novel, chromosome-specific oligonucleotides that are highly visible when labeled and used as probes for chromosomal analysis. Such highly visible probes would generally allow for greater detection sensitivity in chromosome-based diagnostic methods and, in some applications, would allow for the detection of specific chromosomes without computer-assisted methods for signal enhancement or integration.

**BRIEF SUMMARY OF THE INVENTION**

The present invention is generally directed to chromosome-specific nucleic acid sequences. Various aspects of the invention include, for example, synthetic oligonucleotides and labeled probes comprising the chromosome-specific nucleic acid sequences, methods for selecting such sequences, methods and kits for preparing the oligonucleotides or labeled probes, and methods and kits for detecting a specific human chromosome.

Generally, a synthetic oligonucleotide of the present invention comprises a chromosome-specific sequence consisting of 25-35 contiguous nucleotides perfectly complementary to a repeat sequence (e.g., a centromeric repeat sequence, a pericentromeric repeat sequence, a heterochromatin repeat sequence, a telomeric repeat sequence, an alpha satellite repeat sequence, a beta satellite repeat sequence, a gamma satellite repeat sequence, or a satellite 1, 2, or 3 repeat sequence) on a specific human chromosome (e.g., chromosome 2 or 4, or the Y chromosome), the chromosome-specific sequence being less than 84% identical to a consensus repeat sequence of all human chromosomes. Typically, the synthetic oligonucleotide is less than 84% identical to all other contiguous nucleic acid sequences within the human genome. In some embodiments, the chromosome specific sequence consists of 25-50, 25-45, 25-40, or 25-30 contiguous nucleotides perfectly complementary to a repeat sequence (e.g., a centromeric repeat sequence, a pericentromeric repeat sequence, a heterochromatin repeat sequence, a telomeric repeat sequence, an alpha satellite repeat sequence, a beta satellite repeat sequence, a gamma satellite repeat sequence, or a satellite 1, 2, or 3 repeat sequence).

The synthetic oligonucleotide of the present invention may further comprise a detectable label (e.g., a fluorophore). In some embodiments, the label is attached to the chromosome-specific sequence via a linker, crosslinker, or spacer.

The synthetic oligonucleotide of the present invention may further comprise an oligonucleotide that forms a hairpin structure comprising a stem region and a loop region wherein a plurality of nucleotides within the hairpin structure have an attached detectable label. In some embodiments, the stem region comprises 16-40 nucleotides. In some embodiments, at least two of the nucleotides having an attached detectable label are adjacent. In some embodiments, the nucleotides having an attached detectable label are spaced at least 2, 3, 4, 5, 6, 7, 8, or 9 nucleotides apart. In some embodiments, the label is a fluorophore (e.g., fluorescein, rhodamine, Texas Red, phycoerythrin, Cy3, Cy5, Alexa 532, Alexa 546, Alexa 568, or Alexa 594). In some embodiments, the label is attached to the oligonucleotide that forms a hairpin structure via a linker, crosslinker, or spacer.

The synthetic oligonucleotide of the present invention can further include a G-rich nucleic acid sequence. Typically, the G-rich nucleic acid sequence includes at least about 75% G nucleotides. The G-rich nucleic acid sequence can include, for example, from about 20 to about 100 nucleotides, typically from about 30 to about 40 nucleotides. In some variations of an oligonucleotide comprising the chromosome-specific sequence and the G-rich sequence, the oligonucleotide further includes a third nucleic acid sequence linking the G-rich sequence to the chromosome-specific sequence. The G-rich sequence can be, for example, a terminal sequence *(e.g.,* a G-rich sequence is attached to the 5' end of the chromosome-specific sequence or a G-rich sequence is attached to the 3' end of the chromosome-specific sequence).

A labeled probe of the present invention generally includes an oligonucleotide as described herein and a detectable label. The label can be, for example, a direct label. Particularly suitable direct labels include fluorophores such as, *e.g.,* fluorescein, rhodamine, Texas Red, phycoerythrin, Cy3, Cy5, Alexa 532, Alexa 546, Alexa 568, or Alexa 594. Alternatively, the label can be an indirect label.

In certain embodiments, where the labeled probe includes a G-rich sequence as described herein, the labeled probe further includes a platinum labeling complex attached to a G nucleotide within the G-rich sequence, the platinum labeling complex comprising the detectable label. In some of these embodiments, the label is the platinum of the platinum complex. Further, in certain variations, the labeled probe includes a plurality of platinum labeling complexes *(e.g.,* at least three), each complex attached to a different G nucleotide within the G-rich nucleic acid sequence. Typically, the platinum complex is attached to the N7 group of the G nucleotide.

In some aspects, the present invention further provides a probe cocktail composition. Generally, the probe cocktail composition includes a plurality of different labeled probes, each different labeled probe being a probe as described herein and having (a) a different chromosome-specific sequence and (b) a different detectable label that is distinguishable from each of the other detectable labels. In some of these embodiments, each detectable label is a

fluorophore having a spectrally distinguishable emission wavelength.

Also provided are methods of identifying a chromosome-specific probe sequence. In some embodiments, the method generally includes (a) comparing repeat sequences of a specific human chromosome (e.g., chromosome 1, 2, 3, 4, 5, 6, 7, 8, 9, 10, 11, 12, 13, 14, 15, 16, 17, 18, 19, 20, 21, 22, the X chromosome or the Y chromosome) with a consensus repeat sequence of all human chromosomes, and (b) identifying a nucleic acid sequence consisting of 25-35 contiguous nucleotides within the specific repeat sequence and having less than 84% identity to the consensus repeat sequence, thereby identifying a potential chromosome-specific probe sequence. In some embodiments, the nucleic acid sequence of step (b) consists of 25-50, 25-45, 25-40, or 25-30 contiguous nucleotides within the specific repeat sequence. In some embodiments, the repeat sequences comprises a centromeric repeat sequence, a pericentromeric repeat sequence, a heterochromatin repeat sequence, or a telomeric repeat sequence. In some embodiments, the consensus repeat sequence comprises an alpha satellite repeat sequence, a beta satellite repeat sequence, a gamma satellite repeat sequence, a telomeric repeat sequence, or a satellite 1, 2, or 3 repeat sequence. In other embodiments, the method generally includes (a) comparing centromeric repeat sequences of a specific human chromosome *(e.g.,* chromosome 1, 2, 3, 4, 5, 6, 7, 8, 9, 10, 11, 12, 13, 14, 15, 16, 17, 18, 19, 20, 21, 22, the X chromosome or the Y chromosome) with alpha-monomer repeat sequences of all human chromosomes, and (b) identifying a nucleic acid sequence consisting of 25-35 contiguous nucleotides within the specific centromeric repeat sequence and having less than 84% identity to all other alpha-monomer repeat sequences, thereby selecting a chromosome-specific probe sequence. In some embodiments, the nucleic acid sequence of step (b) consists of 25-50, 25-45, 25-40, or 25-30 contiguous nucleotides within the specific repeat sequence. These methods can further include preparing an oligonucleotide probe comprising the selected chromosome-specific nucleic acid sequence.

In some embodiment, the methods comprise (e) labeling the chromosome-specific probe sequence with a detectable label; (f) *performing in situ* hybridization on human metaphases or interphases using the labeled probe (g) detecting the signal associated with the label, wherein chromosome specific probe sequence that produces a signal at least two fold higher upon hybridization is selected as a chromosome specific probe. In certain variations of the methods for selecting a chromosome-specific probe sequence, the method includes identifying a plurality of nucleic acid sequences as in step (b), thereby selecting a plurality of chromosome-specific probe sequences. Some of these variations further include preparing a plurality of labeled oligonucleotide probes, each probe comprising a detectable label and a different chromosome-specific nucleic acid sequence, each different sequence being one of the chromosome-specific sequences selected in (b); performing, individually with each of the different oligonucleotide probes, *in situ* hybridization on human metaphases or interphases; detecting a signal associated with the label for each of the hybridized probes; and selecting one or more of the oligonucleotide probes based on the degree of detected signal.

In other embodiments of the methods for selecting a chromosome-specific probe sequence, the method further includes (c) comparing the nucleotide sequences of the nucleic acid sequences identified in (a) with all other contiguous nucleotide sequences within the human genome; and (d) retaining any sequences that have less than 84% identity to another contiguous nucleotide sequence within the human genome or discarding any sequences that have less than 16% mismatch to another contiguous nucleotide sequence within the human genome, wherein such sequences are identified as potential chromosome specific probe sequences. In some embodiments the methods further comprise preparing a plurality of labeled oligonucleotide probes, each probe comprising a detectable label and a different chromosome-specific nucleic acid sequence, each different sequence being one of the chromosome-specific sequences not discarded in (d); performing, individually with each of the different oligonucleotide probes, *in situ* hybridization on human metaphases or interphases; detecting a signal associated with the label for each of the hybridized probes; and selecting one or more of the oligonucleotide probes based on the degree of detected signal.

Also provided are methods for preparing a labeled probe. In some embodiments, the methods comprise directly attaching a label *(e.g.,* a fluorophore) to the 5' or 3' end of a chromosome-specific oligonucleotide. In some embodiments, the methods comprise attaching a label *(e.g.,* a fluorophore) to a chromosome-specific oligonucleotide comprising a hairpin structure. In some embodiments, the methods generally include (1) attaching at least one G nucleotide of a G-rich nucleic acid sequence to a platinum complex, where said platinum complex comprises a detectable label or is capable of attachment to the detectable label, and (2) attaching the G-rich sequence to a chromosome-specific nucleic acid sequence consisting of 25-35 contiguous nucleotides perfectly complementary to a repeat sequence on a specific human chromosome, where the chromosome-specific sequence is less than 84% identical to a consensus repeat sequence *(e.g.,* an alpha satellite repeat sequence, a beta satellite repeat sequence, a gamma satellite repeat sequence, a telomeric repeat sequence, or a satellite 1, 2, or 3 repeat sequence) of all human chromosomes. Step (2) can be performed before step (1) or *vice versa.* In some embodiments, the chromosome-specific nucleic acid sequence of step (2) consists of 25-50, 25-45, 25-40, or 25-30 contiguous nucleotides perfectly complementary to a repeat sequence on a specific human chromosome. In certain variations, the platinum complex is capable of attachment to the detectable label and the method further includes attaching the platinum complex to the detectable label. Further, the method can include attaching each of a plurality of G nucleotides within said G-rich nucleic acid sequence to a separate platinum complex. Typically, the G-rich nucleic acid sequence includes at least about 75% G nucleotides. The G-rich nucleic acid

sequence can include, for example, from about 20 to about 100 nucleotides, typically from about 30 to about 40 nucleotides. The G-rich sequence can be, for example, a terminal sequence *(e.g.,* a G-rich sequence is attached to the 5' end of the chromosome-specific sequence or a G-rich sequence is attached to the 3' end of the chromosome-specific sequence).

A method of detecting a specific human chromosome, in accordance with the present invention, generally includes contacting a preparation of human chromosomes with a labeled probe as described herein; incubating the chromosome preparation and the probe under conditions sufficient to allow the probe to hybridize to, if present, a specific human chromosome having a repeat sequence perfectly complementary to the chromosome-specific sequence of the probe; and detecting the label to detect the specific human chromosome, if present. In some variations, the method is a method for identifying a chromosomal abnormality. The detection of the label can be accomplished using any methods known in the art, including, for example, microscopic imaging using a microscope and a CCD camera. In some embodiments, the detection of the label involved image scanning microscopy using a computer and a CCD camera. In typical embodiments, the chromosome preparation includes a human cell and the hybridization step is performed *in situ* on chromosomes within the cell.

In certain embodiments, the method includes contacting the human cell with a plurality *(e.g.,* 2, 3, 4, 5, 6, 7, 8, 9, 10, 11, 12, 13, 14, 15, 16, 17, 18, 19, 20 or more) of different labeled probes, each different labeled probe having (a) a different chromosome-specific sequence and (b) a different detectable label that is distinguishable from each of the other detectable labels. In some such embodiments, each detectable label is a fluorophore having a spectrally distinguishable emission wavelength.

Also provided are various kits for preparation of a labeled probe, as well as kits for detection of a target chromosome. In some embodiments, the kits comprise a first container providing an oligonucleotide comprising a chromosome-specific sequence and a second container providing a label *(e.g.* a fluorophore). In some embodiments the oligonucleotide comprising the chromosome-specific sequence further comprises a hairpin structure. In some embodiments, the kits comprise a first container providing an oligonucleotide comprising a chromosome-specific sequence and a G-rich nucleic acid sequence as described herein; and a second container providing a platinum complex. The platinum complex includes a first leaving group, characterized by its ability to be replaced by a G nucleotide; and a group selected from (i) a detectable label, (ii) a linker attached to the detectable label, (iii) a linker capable of attachment to the detectable label; and (iv) a second leaving group, characterized by its ability to be replaced by at least one of (i), (ii), and (iii). In some embodiments, the platinum complex includes the linker of (iii) and the kit further includes a third container providing the detectable label. In some alternative embodiments, the platinum complex includes the second leaving group and the kit further includes a third container providing (i), (ii), or (iii).

Kits for detection of a target chromosome in a cell generally include a first container providing a labeled probe as described herein. In certain variations in which the probe has an indirect label, the kit further includes a second container providing a secondary agent for detecting the indirect label.

## BRIEF DESCRIPTION OF THE DRAWINGS

Figure 1 depicts four exemplary labeling strategies for chromosome-specific probes comprising hairpin structures. The labeled nucleotides are set forth in bold. Fig. 1A shows label spacing every third nucleotide, with the exception of the loop structure. Fig 1B shows a minimally labeled stem-loop structure, where labeling density should not impact fluorophore coupling, or induce quenching. Fig. 1C shows labeling every sixth nucleotide, and the loop apex. Labeled nucleotides occupy opposite positions on the stem helix. Fig. 1D shows labeling of one stem strand only, in addition to the loop structure. Linear label position on the stem helix places amino groups 90° apart, assuming β-DNA formation.

## DETAILED DESCRIPTION OF THE INVENTION

### I. Introduction

The present invention provides compositions and methods generally directed to the use of smaller, chromosome-specific oligonucleotide probes having high detection sensitivity and specificity. Chromosome-specific oligonucleotides as described herein can be labeled by any of a variety of techniques to yield probes that are highly visible, permitting, in certain embodiments, the detection of target human chromosomes (e.g., chromosome 1, 2, 3, 4, 5, 6, 7, 8, 9, 10, 11, 12, 13, 14, 15, 16, 17, 18, 19, 20, 21, 22, the X chromosome or the Y chromosome) without the need for computer-assisted digital enhancement or integration.

**II. Definitions**

Unless defined otherwise, all technical and scientific terms used herein have the same meaning as commonly understood by one of ordinary skill in the art to which this invention belongs. Although any methods and materials similar to those described herein can be used in the practice or testing of the present invention, only exemplary methods and materials are described. For purposes of the present invention, the following terms are defined below.

A "chromosome" is a chromatin complex comprising all or a portion of the genome of a cell. The genome of a cell is often characterized by its karyotype, which is the collection of all the chromosomes that comprise the genome of the cell. The genome of a cell can comprise one or more chromosomes. The human genome comprises 23 pairs of chromosomes, including chromosomes 1, 2, 3, 4, 5, 6, 7, 8, 9, 10, 11, 12, 13, 14, 15, 16, 17, 18, 19, 20, 21, 22, and a pair of X and Y chromosomes.

"Interphase" refers to a stage in cell division in which chromosomes are elongated and entwined with one another. The term "interphases" refers to interphase chromosomes.

"Metaphase" refers to any stage of cell division in which the chromosomes are condensed or contracted and are distinguishable from one another. The term "metaphases" refers to metaphase chromosomes.

"Repeat sequence" or "repetitive sequence" refers to noncoding tandemly repeated nucleotide sequences in the human genome including, e.g., repeat sequences from the alpha satellite, satellite 1, satellite 2, satellite 3, the beta satellite, the gamma satellite and telomeres. Repeat sequences are known in the art and are described in e.g., (Allshire et al., Nucleic Acids Res 17(12): 4611-27 (1989); Cho et al., Nucleic Acids Res 19(6): 1179-82 (1991); Fowler et al., Nucleic Acids Res 15(9): 3929 (1987); Haaf et al., Cell 70(4): 681-96 (1992); Lee et al., Chromosoma 109(6): 381-9 (2000); Maeda and Smithies, Annu Rev Genet 20: 81-108 (1986); Meyne and Goodwin, Chromosoma 103(2): 99-103 (1994); Miklos (1985). Localized highly repetitive DNA sequences in vertebrate genomes. Molecular evolutionary genetics. I. J. R. Macintyre. NY, Plenum Publishing Corp.: 241-321 (1985); Tagarro et al., Hum Genet 93(2): 125-8 (1994); Waye and Willard, PNAS USA 86(16): 6250-4 (1989); and Willard and Waye, J Mol Evol 25(3): 207-14 (1987). The repeat sequences are located at, e.g., the centromeric, pericentromeric, heterochromatic, and telomeric regions of chromosomes. Consensus repeat sequences are described in, e.g. Willard and Waye, J Mol Evol 25(3): 207-14 (1987) and Tagarro et al., Hum Genet 93(2): 125-8 (1994). Vissel and Choo, Nucleic Acids Res. 15(16): 6751-6752 (1987), Cho et al., Nucleic Acids Res 19(6): 1179-82 (1991).

The term "nucleotide," in addition to referring to the naturally occurring ribonucleotide or deoxyribonucleotide monomers, shall herein be understood to refer to related structural variants thereof, including derivatives and analogs, that are functionally equivalent with respect to the particular context in which the nucleotide is being used *(e.g.,* linkage to a platinum complex, hybridization to complementary base, or linkage of two non-adjacent nucleic acid sequences), unless the context clearly indicates otherwise. Accordingly, nucleotides can include, for example, nucleotides comprising naturally occurring bases *(e.g.,* A, G, C, or T) or modified bases *(e.g.,* 7-deazaguanosine, or inosine).

The term "nucleic acid" refers to a polymer having multiple nucleotide monomers. A nucleic acid can be single- or double-stranded, and can be DNA (cDNA or genomic), RNA, synthetic forms, and mixed polymers, and can also be chemically or biochemically modified or can contain non-natural or derivatized nucleotide bases. Such modifications include, for example, methylation, substitution of one or more of the naturally occurring nucleotides with an analog, internucleotide modifications such as uncharged linkages *(e.g.,* methyl phosphonates, phosphotriesters, phosphoamidates, carbamates, and the like), charged linkages *(e.g.,* phosphorothioates, phosphorodithioates, and the like), pendent moieties *(e.g.,* polypeptides), intercalators *(e.g.,* acridine, psoralen, and the like), chelators, alkylators, and modified linkages *(e.g.,* alpha anomeric nucleic acids, and the like). Also included are synthetic molecules that mimic nucleic acids in their ability to bind to a designated sequence via hydrogen bonding and other chemical interactions. Typically, the nucleotide monomers are linked via phosphodiester bonds, although synthetic forms of nucleic acids can comprise other linkages *(e.g.,* peptide nucleic acids (also referred to herein as "PNAs"), such as described in Nielsen et al., Science 254, 1497-1500, 1991). nucleic acids can also include, for example, conformationally restricted nucleic acids *(e.g.,* "locked nucleic acids" or "LNAs," such as described in Nielsen et al., J. Biomol. Struct. Dyn. 17:175-91, 1999). "Nucleic acid" do not refer to any particular length of polymer and can, therefore, be of substantially any length, typically from about six (6) nucleotides to about $10^9$ nucleotides or larger. In the case of a double-stranded polymer, "nucleic acid" can refer to either or both strands.

The term "oligonucleotide" refers to a subset of nucleic acids of from about 6 to about 175 nucleotides in length. Oligonucleotides can be synthesized using known methods (e.g., using an automated oligonucleotide synthesizer such as, for example, those manufactured by Applied BioSystems (Foster City, CA)).

The term "sequence," in reference to a nucleic acid, refers to a contiguous series of nucleotides.

The term "chromosome-specific nucleic acid sequence," as used herein, refers to a nucleic acid sequence that is specific to a particular chromosome within the genome of the cell. A nucleic acid sequence is specific to a particular chromosome within the genome where that sequence is perfectly complementary to a segment of nucleotides on that particular chromosome, but where the perfectly complementary nucleotide segment is absent from most or all other

chromosomes of the genome.

A "chromosome-specific nucleic acid" or "chromosome-specific oligonucleotide," as used herein, means a nucleic acid or oligonucleotide comprising a chromosome-specific nucleic acid sequence.

The term "hairpin structure" or "hairpin sequence" refers to a nucleic acid having a double-stranded "stem" region and a single-stranded "loop" region, in which the stem region and loop region are formed from a single strand of nucleic acid having (1) two regions that are mutually, substantially complementary so as to form the double-stranded sequence comprising the stem *(i. e.,* via complementary base pairing) and (2) interposed between the two mutually complementary regions, a third region that forms the loop. Hairpin structures are described in, for example, Varani, Annu. Rev. Biophys. Biomol. Struct. 24:379-404, 1995. Hairpin structures may comprise modified nucleotides, including *e.g.,* amino-modified nucleotides, to provide reactive functional groups for attachment of a label such as a fluorophore.

The term "adjacent," in reference to two nucleic acid sequences *(e.g.,* a chromosome-specific nucleic acid sequence and a hairpin sequence or a chromosome-specific nucleic acid sequence and a G-rich nucleic acid sequence), means that the two sequences are non-overlapping (not sharing one or more nucleotides defining the two sequences) and are not separated by an intervening molecule *(i.e.,* not separated by a "linker" as defined below).

The term "G-rich nucleic acid sequence," as used herein, refers to a particular region or part of a synthetic oligonucleotide or probe of the present invention that links a chromosome-specific nucleic acid sequence to one or more platinum complexes. "G-rich nucleic acid sequence" means a sequence of nucleic acid comprising at least about 50%, typically at least about 60% and more typically at least about 75% guanine nucleotides. "Guanine nucleotides," as used in the context of a G-rich nucleic acid sequence, means a nucleotide comprising a naturally occurring guanine base or, alternatively, a modified guanine base or guanine analog that having a acceptor group suitable for linkage to a platinum complex. Typically, the G-rich nucleic acid sequence is a part or region of the probe that is mutually exclusive from the target-binding element, *i.e.,* the G-rich sequence is typically not designed to bind specifically to a target. G-rich nucleic acid sequences in accordance with the present invention are typically at least 20 nucleotides in length. In various embodiments, the G-rich nucleic acid sequence is at least 30 nucleotides, at least 40 nucleotides, or at least 50 nucleotides in length. In other, non-mutually exclusive embodiments, the G-rich nucleic acid sequence is no more than 70, no more than 80, nor more than 90, or no more than 100 nucleotides in length.

A "terminal" G-rich nucleic acid sequence, in the context of probes having a nucleic acid target-binding sequence, means that the G-rich sequence is a terminal region of the probe, *i.e.,* either the most 5' region or the most 3' region.

A "linker," in the context of attachment of a two molecules (whether monomeric or polymeric), means a molecule (whether monomeric or polymeric) that is interposed between and adjacent to the two molecules being attached. A "linker" can be used to attach, *e.g.,* chromosome-specific nucleic acid sequence and a label, a chromosome-specific nucleic acid sequence and a hairpin structure, a chromosome-specific nucleic acid sequence and a G-rich nucleic acid sequence, or a detectable label and a platinum atom in a platinum complex. The linker can be a nucleotide linker (*i.e.,* a sequence of the nucleic acid that is between and adjacent to the non-adjacent sequences) or a non-nucleotide linker. Thus, for example, in the specific context of attachment of a chromosome-specific nucleic acid sequence and a G-rich nucleic acid sequence that are non-adjacent and non-overlapping, a "linker" means a molecule that is interposed between and adjacent to the non-adjacent chromosome-specific and G-rich sequences.

A "probe," as used herein, refers to a nucleic acid capable of binding to a complementary target nucleic acid. As described further herein, a probe binds to a complementary target site in a chromosome. Typically, the probe is a labeled probe, having one or more detectable labels to permit the detection of the probe following hybridization to its complementary target.

A "platinum complex" or "platinum-containing compound," as used herein, means a molecule comprising a multivalent platinum atom and that has formed or has the ability to form an adduct with a guanine nucleotide by reacting with a guanine nucleotide acceptor, thereby linking the platinum complex to at least one guanine nucleotide of a G-rich nucleic acid sequence. In typical embodiments of the present invention, a platinum complex is tetravalent or hexavalent. "Platinum labeling complex" or "platinum labeling compound" means a platinum complex that comprises a detectable label. Platinum-containing compounds suitable for use in accordance with the present invention are described, for example, in International Patent Publication No. WO 92/01699; U.S. Patent No. 5,714,327; and U.S. Patent No. 6,825,330.

As used herein, the term "adduct" refers to the complex formed by the co-ordination of the platinum atom in a platinum complex to an atom of a G nucleotide. In an adduct as used herein, the platinum atom directly participates in the binding of the platinum complex to the G nucleotide.

As used herein, the term "leaving group" refers to a moiety that can be displaced from a platinum atom in a platinum complex as described herein, in favor of an acceptor molecule to be attached to the platinum atom under appropriate conditions. For example, a leaving group can be characterized by its ability to be replaced by a G nucleotide of a G-rich nucleic acid sequence, by a detectable label, or by a linker attached to or capable of attachment to a detectable label. The selection of leaving groups is within the ability of one skilled in the art, through comparison of the electronegativities of the candidate leaving group and the acceptor molecule (that is, a relatively more electronegative group on the candidate acceptor molecule will tend to displace a relatively less electronegative leaving group). For example,

suitable leaving groups for attachment of the platinum complex to a G-rich nucleic acid sequence would include groups that would permit a bond between the platinum ion and a guanine nucleotide acceptor to be formed under appropriate conditions. Leaving groups include, for example, F, $NO_2$, Ots, $SOPO_4$, Cl, Br, I, CN, N3, NR3, OAr, OR, SR, $SO_2R$ and $NH_2$, where R is an organic residual group and Ar is an aryl organic residual group. Others include, for example, sulfate, nitrate, phosphate, carbonate and lower alkyl derivatives thereof *(e.g.,* ethylnitrate, propylnitrate, and the like). Appropriate conditions for displacement of a leaving group are generally known in the art and can vary depending on the particular leaving group and acceptor molecule. In certain variations, appropriate conditions includes solutions containing molecules having "soft" ligands such as heavier halogens, phosphines, sulfides, nitrogen compounds such as amines, adenine, adenosine and its derivatives, guanine, guanosine and its derivatives, DNA, RNA, PNA, alkenes, alkynes and other $\pi$-compounds, heated to 15-100° C between 0.5 to 120 minutes.

The term "detectable label," as used herein in the context of a probe comprising a label, refers to an agent that is capable of detection such that the presence of the corresponding probe, to which it is bound, can be detected. Labels can be indirect or direct. An "indirect label" is a specifically bindable molecule (a "ligand") that is detected using a labeled secondary agent (a "ligand-binding partner") that specifically binds to the indirect label. Conversely, a "direct label" is detected without a ligand-binding partner interaction. For indirect labels, the secondary agent typically has a direct label, or, alternatively, the secondary agent can also be labeled indirectly. Typical "direct labels" include, for example, fluorophores *(e.g.,* fluorescein, rhodamine, or phthalocyanine dyes), chromophores *(e.g.,* phycobiliproteins), luminescers *(e.g.,* chemiluminescers and bioluminescers), lanthanide chelates *(e.g.,* complexes of $Eu^{3+}$ or $Tb^{3+}$), enzymes *(e.g.,* alkaline phosphatase), cofactors, and residues comprising radioisotopes such as, *e.g.,* $^{3}H$, $^{35}S$, $^{32}P$, $^{125}I$, and $^{14}C$. Typical indirect labels include, *e.g.,* haptens, biotin, or other specifically bindable ligands.

A "hapten" means an isolated epitope, *i.e.,* a molecule having an antigenic determinant. Examples of haptens include dinitrophenol (DNP), digoxigenin, biotin, and various fluorophores or dyes *(e.g.,* fluorescein, Alexa 405/Cascade blue, Alexa 488, Bodiby FL, Dansyl, Oregon Green, Lucifer Yellow, Tetramethylrhodamine/Rhodamine Red, and Texas Red). As an indirect label, a hapten is typically detected using an anti-hapten antibody as the ligand-binding partner. However, a hapten can also be detected using an alternative ligand-binding partner *(e.g.,* in the case of biotin, anti-biotin antibodies or streptavidin, for example, can be used as the ligand-binding partner). Further, in certain embodiments, a hapten can also be detected directly *(e.g.,* in the case of fluorescein, an anti-fluorescein antibody or direct detection of fluorescence can be used).

The present invention provides compositions and methods generally directed to the use of smaller, chromosome-specific oligonucleotide probes having high detection sensitivity and specificity. Chromosome-specific oligonucleotides as described herein can be labeled by any of a variety of techniques to yield probes that are highly visible, permitting, in certain embodiments, the detection of target human chromosomes without the need for computer-assisted digital enhancement or integration.

## III. Chromosome-Specific Oligonucleotides, Probes, and Probe Cocktails

Accordingly, in certain aspects, the present invention provides novel synthetic oligonucleotides having a chromosome-specific sequence. Such synthetic oligonucleotides are useful, for example, in the preparation of labeled probes for detection of specific target chromosomes including, e.g., chromosome 2 or 4, or the Y chromosome. Generally, a synthetic oligonucleotide of the present invention comprises a chromosome-specific sequence consisting of 25-35 contiguous nucleotides perfectly complementary to a repeat sequence on a specific human chromosome. Typically, the chromosome-specific sequence is less than 84% identical to a consensus repeat sequence *(e.g.,* an alpha satellite repeat sequence, a beta satellite repeat sequence, a gamma satellite repeat sequence, a telomeric repeat sequence, or a satellite 1, 2, or 3 repeat sequence) of all human chromosomes. In certain embodiments, the chromosome-specific sequence is less than 85%, less than 84%, less than 80%, less than 78%, less than 75%, less than 73%, or less than 70% identical to the consensus repeat sequence. Preferably, the synthetic oligonucleotide is less than 84% identical to all other contiguous nucleic acid sequences within the human genome.

In accordance with the present invention, the chromosome-specific sequence of the synthetic oligonucleotide is specific for a particular human chromosome. In certain variations, the chromosome-specific sequence is specific for human chromosome 2 or 4 or the Y chromosome. Exemplary chromosome 2, chromosome 4, and Y chromosome -specific nucleic acid sequences are set forth in Table 1.

**Table 1.** Exemplary Chromosome-Specific Nucleic Acid Sequences.

| SEQ ID NO: | NAME | SEQUENCE (5'-3') |
|---|---|---|
| 1 | Y1 | CCAGTCGAATCCATTCGAGTACATACC |
| 2 | Y2 | CCTTTTGAATCCATTCCATTGGAGTCC |

(continued)

| SEQ ID NO: | NAME | SEQUENCE (5'-3') |
|---|---|---|
| 3 | Y3 | ATTCATTGCATTCCGTTTCATGAAATTCGA |
| 4 | Y4 | CTGCATACAATTTCACTCCATTCGTTCCCA |
| 5 | Y5 | TCCATTGGAGTCAATTCCTTTCGACACCCA |
| 6 | Y6 | TTGATCCTATTTTATTAAATTGCATTCTAT |
| 7 | 2.1.1 | GTGCGCCCTCAACTAACAGTGTTGAAGCTT |
| 8 | 2.2.2 | GAAACGGGATTGTCTTCATATAAACTCTAG |
| 9 | 2.5.1 | GTATCTTCCAATAAAAGCTAGATAGAAGCA |
| 10 | 2.6.1 | ATGTCAGAAACTTTTTCATGATGTATCTAC |
| 11 | 2.7.3 | TATGTGTGATGTGCGCCCTCAACTAAGAGT |
| 12 | 2.8.4 | TCTCAGAAGCTTCATTGGGATGTTTCAATT |
| 13 | 2.10.1 | GGAATACGGTGATAAAGGAAATATCTTCCA |
| 14 | 4.3.2 | TCTTTGTGTTGTGTGTACTCATGTAACAGT |
| 15 | 4.6.2 | TTTCTGCCCTACCTGGAAGCGGACATTTCG |
| 16 | 4.7.5 | GGTTATCTTCATATAAAATCCAGACAGGAG |
| 17 | 4.10.2 | CGGCACTACCTGGAAGTGGATATTTCGAGC |
| 18 | 4.18.7 | TCTGCACTACCTGGAAGAGGCCATTTCGAG |
| 19 | 4.22.10 | CCTACGGGGAGAAAGGAAATATCTTCAAAT |

In certain embodiments, the oligonucleotide further comprises a hairpin structure comprising a stem region and a loop region, wherein a plurality of nucleotides within the hairpin structure have an attached detectable label such as, e.g., a fluorophore. The hairpin structure may comprise modified nucleotides having a reactive group that facilitates attachment of a label to the hairpin. Exemplary reactive groups are set forth in Table 2 below. The hairpin region typically includes from about 18 to about 50 nucleotides, more typically from about 25 to about 40 nucleotides. Formation of the hairpin structure is accomplished via design of two subregions having substantial anti-parallel complementarity, i.e., sufficient complementarity running in opposite directions to specifically hybridize under stringent conditions, thereby forming a stem region. Between the substantially mutually complementary regions is interposed a non-complementary sequence, which does not hybridize intramolecularly and therefore has as a single-stranded loop configuration upon formation of the double-stranded stem region. The number and base composition of nucleotides in the loop region are selected to allow the mutually complementary subregions of the stem to hybridize. For example, the loop region is designed to avoid substantial complementarity with either strand of the stem region and typically has from 3 to 20 nucleotides, more typically from 3 to 10 nucleotides, and most typically from 5 to 8 nucleotides. As indicated *supra*, the number and base composition of nucleotides in the stem region are selected for substantial complementarity of two regions running in opposite directions such that a double-stranded hybrid of a desired relative stability is formed under stringent hybridization conditions. Typically, the stem region typically has from 14 to 46 nucleotides (*i.e.,* 7-23 nucleotides for each strand of the stem), more typically from 16 to 40 nucleotides (*i.e.,* 8-20 nucleotides for each strand of the stem), and most typically from 20 to 32 nucleotides *(i.e.,* 10-16 nucleotides for each strand of the stem). Exemplary hairpin structures are set forth in Figure 1.

In certain embodiments, particularly variations relating to platinum-complex-based labeling of chromosome-specific probes, the synthetic oligonucleotide further includes a G-rich nucleic acid sequence. Preferably, the G-rich nucleic acid sequence comprises at least about 75% G nucleotides. In addition, the G-rich nucleic acid sequence typically includes from about 20 to about 100 nucleotides, more typically from about 30 to about 60 nucleotides, from about 30 to about 50 nucleotides, from about 30 to about 40 nucleotides, or from about 20 to about 40 nucleotides.

A chromosome-specific nucleic acid sequence can be directly linked to a G-rich sequence. In other variations comprising a G-rich sequence, the chromosome-specific oligonucleotide further includes a linker interposed between and adjacent to the G-rich and chromosome-specific sequences. The linker is typically one or more nucleotides, including non-natural or derivatized nucleotides. The number and composition of bases for nucleotide linkers are selected to not interfere with hybridization of the target-binding region to its target. Nucleotide linkers are typically from 1-20 nucleotides

in length, more typically from 1-10 and even more typically from 1-5 nucleotides in length. Thus, a third nucleic acid sequence can serve as the linker. Alternatively, non-nucleotide linkers of various lengths can be used. Suitable non-nucleotide linkers are known in the art and include, for example, Spacer Phosphoramidites C3, 9, and 18 (Glenn Research, #10-1913, 10-1909, or 10-1918, respectively). The G-rich sequence can be linked at either 5' or 3' end of the nucleic acid target-binding sequence. Typically, the G-rich sequence is a terminal sequence. Typically, the G-rich sequence is a terminal sequence, attached to either the 5' or 3' end of the chromosome-specific sequence. In some embodiments, each end of the chromosome-specific sequence is attached, either directly or via a linker, to a G-rich sequence.

Labeled oligonucleotide probes in accordance with the invention generally comprise a chromosome-specific oligonucleotide as described above and a detectable label. The detectable label can be direct or indirect. Labels suitable for use according to the present invention are known in the art and generally include any molecule that, by its chemical nature and whether by direct or indirect means, provides an identifiable signal allowing detection of the probe. Preferred direct labels include fluorophores such as, for example, fluorescein, rhodamine, Texas Red, phycoerythrin, phthalocyanine dyes *(e.g.,* Cy3 or Cy5), Alexa 532, Alexa 546, Alexa 568, and Alexa 594. Other direct labels can include, for example, radionuclides and enzymes such as, e.g., alkaline phosphatase, horseradish peroxidase, or β-galactosidase. Alternatively, indirect labels can be used. For example, the indirect label can be biotin, which can be detected using, for example, labeled streptavidin (e.g., streptavidin conjugated to fluorescein). In other embodiments, the indirect label is a hapten which is detected using an anti-hapten antibody. Typical haptens suitable for use according to the present invention include, *e.g.,* biotin, digoxigenin, dinitrophenol (DNP), and fluorophores or dyes such as, for example, fluorescein, Alexa 405/Cascade blue, Alexa 488, Bodipy FL, Dansyl, Oregon Green, Lucifer Yellow, Tetramethylrhodamine/Rhodamine Red, and Texas Red.

In the case of fluorescent labels, fluorophores are not to be limited to single species organic molecules, but include inorganic molecules, multi-molecular mixtures of organic and/or inorganic molecules, crystals, heteropolymers, and the like. For example, CdSe-CdS core-shell nanocrystals enclosed in a silica shell can be easily derivatized for coupling to a biological molecule (Bruchez et al., Science, 281:2013-2016, 1998). Similarly, highly fluorescent quantum dots (zinc sulfide-capped cadmium selenide) have been covalently coupled to biomolecules for use in ultrasensitive biological detection (Warren and Nie, Science, 281: 2016-2018, 1998).

In certain embodiments, where the labeled probe includes a G-rich sequence as described herein, the labeled probe can further include a platinum labeling complex attached to a G nucleotide within the G-rich sequence, the platinum labeling complex having a detectable label. In typical variations, the platinum labeling complex will have the formula $\{Pt^{II}(w)(x)(y)(z)\}$ or $\{Pt^{IV}(u)(v)(w)(x)(y)(z)\}$, shown structurally below as Formulas (1) and (2), respectively:

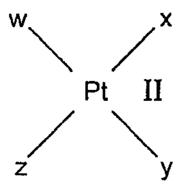

(1)

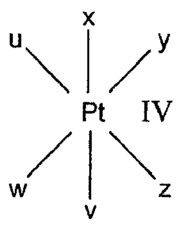

(2)

In Formulas (1) and (2), u, v, w, x, y and z represent ligands to the platinum atom. Formula (1) represents a tetravalent platinum compound having a square planar, tetragonal structure. Formula (2) represents a hexavalent compound having octahedral structure. In these structures, no more than one ligand is a guanine nucleotide of a G-rich nucleic acid sequence. Typically, where the platinum ion itself does not serve as the label, at least one of the ligands represents a detectable label or, alternatively, a stabilizing substituent attached to the detectable label. Optionally, the detectable label is attached to the platinum ion or stabilizing substituent via a linker. Examples of suitable linkers are described further below. The remaining ligands are stabilizing substituents, and maybe the same as or different from one another. Two or more of the ligand sites may be interconnected by the stabilizing ligand(s), preferably covalently, to form a stabilizing bridge as desired. Preferred interconnected groups include aliphatic polyamines. For example, particularly suitable interconnected groups of ligands include aliphatic diamines *(e.g.,* ethylenediamine ($-NH_2-CH_2-CH_2-H_2N-$) or cycloaliphatic diamines), with terminal nitrogens occupying two ligand sites when bound to the

platinum atom. Suitable platinum labeling complexes for linkage of detectable labels to G nucleotides are also described in, *e.g.,* International Patent Publication WO 92/01699; WO 96/35696; WO 98/15564; U.S. Patent No. 5,714,327; and U.S. Patent No. 6,825,330.

In preferred embodiments, multiple G-nucleotides within the G-rich sequence are each linked to a separate platinum labeling complex. In these embodiments, each platinum complex will typically have the same detectable label, although different labels can also be used, depending on the particular application. In specific variations, the probe comprises at least two, at least three, at least five, or at least seven platinum labeling complexes. Typically, the platinum complex is attached to the N7 group of the G nucleotide.

As indicated previously, in certain variations, each end of a chromosome-specific sequence is attached, either directly or via a linker, to a G-rich sequence. Labeled probes comprising such oligonucleotides will typically have at least one G-nucleotide of each G-rich sequence attached to a platinum labeling complex. In preferred embodiments, two or more G-nucleotides of each G-rich sequence, more preferably at least three, at least five, or at least seven G nucleotides, are attached to a platinum labeling complex.

The detectable label of a platinum labeling complex can be either direct or indirect, as indicated previously. In certain variations, the platinum atom itself is the detectable label. For example, platinum compounds can be detected directly by using the platinum as a nucleus for depositing silver or other metal crystals. The platinum compound can also serve, *e.g.,* as an electrocatalytic label (for example, a signal can be achieved by measuring chronoamperometrically the current generated by the hydrogen evolution catalyzed by platinum, *see, e.g.,* Hernandez-Santos et al., Anal. Chem. 77:2868-2874, 2005.

In certain aspects of the invention, a plurality of different labeled probes are mixed to provide a probe cocktail composition. Generally, each of the different labeled probes of the composition has a different chromosome-specific sequence and a different detectable label that is distinguishable from each of the other detectable labels. For example, each detectable label can be a fluorophore having a spectrally distinguishable emission wavelength. In some variations, a probe cocktail composition is prepared by mixing one or more chromosome-specific labeled probe(s). Such probe cocktail compositions can be selected, for example, based on relative specificity of various probes, as well as the optimum number of oligonucleotide probe(s) for each cocktail, as determined, *e.g.,* in hybridization assays. In specific embodiments, a probe cocktail composition is a dual color probe cocktail. For example, a dual color, chromosome probe cocktail composition can be prepared, each of the chromosome-specific probes having a fluorophore with a spectrally distinguishable emission wavelength. Suitable fluorophores for use in such dual color probe cocktail compositions include, *e.g.,* Alexa 488 (excitation maximum at 492 nm and emission maximum at 520 nm) and Alexa 546 (excitation maximum at 555 nm and emission maximum at 570 nm)).

## IV. Preparation of the Oligonucleotide and Probe Compositions

### *A. General*

The present invention also provides a method for preparing a synthetic oligonucleotide or labeled probe as described above. A synthetic, chromosome-specific oligonucleotide as described herein can be synthesized using known methods for nucleic acid synthesis *(see, e.g.,* Glick and Pasternak, Molecular Biotechnology: Principles and Applications of Recombinant DNA (ASM Press 1998)). Solution or solid-phase techniques can be used. Synthesis procedures are typically automated and can include, for example, phosphoramidite, phosphite triester, H-phosphate, or phosphotriester methods.

### *B. Preparing Labeled Probes*

Generally, preparing a labeled probe in accordance with the present invention includes providing a chromosome-specific oligonucleotide and attaching a label to the oligonucleotide. Various labels suitable for use with the oligonucleotide probes, as well as techniques for attaching such labels to an oligonucleotide to yield an oligonucleotide probe, are known in the art and can be used in accordance with the present invention. Thus, for example, the probes may be labeled in any of various conventional manners, such as with specific reporter molecules, fluorophores, radioactive materials, or enzymes *(e.g.,* peroxidases, phosphatases).

In some embodiments, oligonucleotide labeling is performed during synthesis or, alternatively, post-synthesis using 5'-end labeling, which involves the enzymatic addition of a labeled nucleotide to the 5'end of the oligonucleotide using a terminal transferase. A single labeled nucleotide can be added by using a "chain terminating" nucleotide; alternatively, non-terminating nucleotides can be used, resulting in multiple nucleotides being added to form a "tail." For synthesis labeling, another common method, labeled nucleotides *(e.g.,* phosphoramidite nucleotides) are incorporated into the oligonucleotide during chemical synthesis. Labels can be added to the 5', 3', or both ends of the oligonucleotide *(see, e.g.,* U.S. Patent No. 5,082,830), or at base positions internal to the ODN.

In some embodiments, the oligonucleotides comprise labeled hairpin structures. The labels may be on the stem portion, loop portion, or both the stem and loop portion of the hairpin structure. In some embodiments, the labels are on the loop portion of the hairpin structure. In some embodiments, the labels are on adjacent nucleotides. In some embodiments, the labels are spaced, 2, 3, 4, 5, 6, 7, 8, 9, 10, or more nucleotides apart. In some embodiments, the labels are on 1, 2, 3, 4, 5, 6, 7, 8, 9, 10, 11, 12 or more nucleotides of the hairpin structure. In an exemplary embodiment, oligonucleotides comprising hairpin structures are incubated with fluorophore-conjugated N-hydroxysuccinamide (NHS) esters which form covalent bonds only with the reactive amine groups on the hairpin portion of the oligonucleotide. Other suitable bonds are set forth in Table 2 below.

Other methods for labeling nucleic acids utilize platinum-based labeling. Such methods include the Universal Linkage System (ULS, Kreatech Biotechnology B.V., Amsterdam, Netherlands). Platinum based labeling methods and their applications are described in, for example, U.S. Patent Nos. 5,580,990, 5,714,327, and 6,825,330; International Patent Publication Nos. WO 92/01699, WO 96/35696, and WO 98/15546; Hernandez-Santoset et al., Anal. Chem. 77:2868-2874, 2005; Raap et al., BioTechniques 37:1-6, 2004; Heetebrij et al., ChemBioChem 4:573-583, 2003; Van de Rijke et al., Analytical Biochemistry 321:71-78, 2003; Gupta et al., Nucleic Acids Research 31:e13, 2003; Van Gijlswijk et al., Clinical Chemistry 48:1352-1359, 2002; Alers et al., Genes, Chromosomes & Cancer 25:301-305, 1999; Wiegant et al., Cytogenetics and Cell Genetics 87:7-52,1999; Jelsma et al., Journal ofNIHResearch 5:82, 1994; Van Belkum et al., BioTechniques 16:148-153, 1994; and Van Belkum et al., Journal of Virological Methods 45:189-200, 1993.

In certain variations of the invention, platinum-based labeling is used in conjunction with a chromosome-specific probe comprising a G-rich nucleic acid sequence. In the case of a labeled probe comprising a G-rich nucleic acid sequence, a method of preparing the probe generally includes attaching at least one G nucleotide of the G-rich sequence to a platinum complex and attaching the G-rich nucleic acid sequence to a chromosome-specific nucleic acid sequence. As indicated previously, a G-rich nucleic acid sequence for preparing a probe of the present invention preferably includes at least about 75% guanine nucleotides and is typically from about 20 to about 100 nucleotides, more typically from about 30 to about 60 nucleotides, from about 30 to about 50 nucleotides, from about 30 to about 40 nucleotides, or from about 20 to about 40 nucleotides. Attachment of the G-rich sequence to the chromosome-specific sequence can be performed before or after attachment of a G nucleotide to the platinum complex.

Various techniques available in the art can be used to attach the G-rich sequence to the chromosome-specific sequence. The G-rich sequence may be attached to the chromosome-specific sequence via a nucleotide or non-nucleotide linker, which can be essentially of any length and composition which do not interfere with interaction of the target-binding element with a corresponding target molecule. The linker can be attached to the 5' or 3' end of the G-rich sequence or, alternatively, to an internal nucleotide. In other variations, the G-rich sequence is attached directly adjacent to the chromosome-specific sequence, without an intervening linker. Typically, the G-rich sequence and chromosome-specific sequence can simply be synthesized as a single oligonucleotide comprising both sequences, using known techniques for nucleic acid synthesis such as described above. In these cases, the G-rich and chromosome-specific sequences are attached as part of the nucleic acid synthesis procedure and can be linked either 5' or 3' to the chromosome-specific sequence, or both 5' and 3' to the chromosome-specific sequence. Alternatively, a G-rich sequence can be attached 5' to a chromosome-specific sequence by, for example, primer extension. In such embodiments, the G-rich sequence includes a 3' priming sequence that is substantially complementary to a 5' region of a nucleic acid comprising a chromosome-specific sequence, or to a region of a nucleic acid sequence located 5' and adjacent to such a nucleic acid. As used herein, "substantially complementary" means that the 3' priming sequence is capable of hybridizing to the 5' region of the nucleic acid comprising a chromosome-specific sequence under hybridization conditions typically used for primer extension. The priming sequence is allowed to hybridize to the substantially complementary region of the nucleic acid comprising the chromosome-specific sequence, or to the nucleic acid sequence adjacent thereto, under conditions suitable for primer extension. Conditions suitable for primer extension are generally known in the art. *(See, e.g.,* Sambrook and Russell, Molecular Cloning: A Laboratory Manual (3rd ed., Cold Spring Harbor Laboratory Press 2001); Ausubel et al., Short Protocols in Molecular Biology (4th ed., John Wiley & Sons 1999).) In yet other embodiments, the G-rich and chromosome specific sequences are first each synthesized separately *(e.g.,* using known methods for oligonucleotide synthesis) and subsequently attached via an appropriate linker.

The platinum complex generally includes a leaving group characterized by its ability to be replaced by the G nucleotide within the G-rich nucleic acid sequence. The platinum complex further includes a detectable label or is capable of attachment thereto. Accordingly, the platinum complex includes a group selected from the following:

(i) a detectable label

(ii) a linker attached to the detectable label;

(iii) a linker capable of attachment to the detectable label; and

(iv) a second leaving group, characterized by its ability to be replaced by at least one of (i), (ii), and (iii) above.

In some embodiments, the detectable label of (i) is the platinum atom itself. In these cases, the platinum complex need not include a separate group comprising a detectable label, nor a linker for attachment thereto.

In typical variations, the platinum complex will have the formula $\{Pt^{II}(w)(x)(y)(z)\}$ or $\{Pt^{IV}(u)(v)(w)(x)(y)(z)\}$, shown structurally as Formulas (1) and (2), *supra.* As indicated previously, in Formulas (1) and (2), u, v, w, x, y and z represent ligands to the platinum atom. In a method for preparing a probe having a G-rich sequence, no more than one ligand is a leaving group, characterized by its ability to be replaced by a guanine nucleotide of a Grich nucleic acid sequence. In certain embodiments in which the platinum atom itself does not serve as the detectable label, at least one of the ligands represents (a) a detectable label; (b) a linker attached to the detectable label; (c) a linker capable of attachment to the detectable label; (d) a leaving group that is characterized by its ability to be replaced by (a), (b), or (c); or (e) a stabilizing substituent comprising any one of (a) through (d). The remaining ligands are stabilizing substituents, and may be the same as or different from one another. Two or more of the ligand sites may be interconnected by the stabilizing ligand(s), preferably covalently, to form a stabilizing bridge as desired. Suitable platinum complexes for linkage of detectable labels to G nucleotides are also described in, *e.g.,* International Patent Publication WO 92/01699; U.S. Patent No. 5,714,327; and U.S. Patent No. 6,825,330.

Linkers for attaching a detectable label to the platinum compound can be, for example, simple alkyl chains or more complex structures comprising aryl groups and/or heteroatoms. In certain embodiments, the linker is at least four atoms long, preferably at least four carbon atoms long, and has at least one heteroatom in that carbon chain. A heteroatom typically confers a certain amount of rigidity on the linker. This rigidity provides an additional assurance that steric factors will not obstruct a convenient linking of a guanine nucleotide and a detectable label via the platinum labeling complex. A particularly suitable heteroatom is an oxygen atom, which positively effects the hydrophilicity of the linker. In some variations, the linker includes no more than 20 carbon atoms in the chain, which is preferably an essentially nonbranched chain, thereby minimizing any steric hindrance.

One property any linker must have is the ability to be joined to a reactive group suitable for the attachment of the detectable label. Non-limiting examples of reactive groups include amines, carboxylate, and thiol groups. Linkers bearing amines, for example, can be reacted with NHS esters of detectable labels *(e.g.,* fluorescent dye-NHS) to generate a platinum labeling complex.

One suitable linker is 1,8-diamino-3,6-dioxaoctane, which is a very flexible compound with a distal primary amine group and a size that makes it very suitable for use as a linker in accordance with the present invention.

Another linker that can be used is an oligolysine or a polylysine. Due to their structure and conformation, these molecules create a convenient environment for an optimal interaction among the detectable label, the guanine nucleotide, and the platinum. An additional advantage of the use of lysine chains as the linker is, that by altering the number of lysine units in the chain, the optimal conditions for specific labels and nucleotides or nucleic acids can be attained. Given a certain application, the skilled person will routinely determine how many lysine units are required for optimum results.

Other non-limiting examples of suitable linkers include the following: $(CH_2)_n$, $(CH_2)_n(CH=CH)_mO(CH=CH)_p(CH_2)_q$, $CO(CH_2)_n(CH=CH)_m(CH_2)_p$, $COAr(CH_2)_n(CH=CH)_m(CH_2)_p$, $NH_2(CH2)_nQ$, $NH_2((CH2)_nO)_m(CH_2)_tQ$, $NH_2(CH_2)_mAr(CH2)_nQ$, where m, n, p, q and t are integers from 0 to 8, inclusive, and m, n, p, q and t are the same or different. In the foregoing exemplary linker formulae, Q is selected from the group consisting of CONH, NHCO, -S-S-, NHCSNH, NHCSO, where Ar is an aryl organic residual group. Certain variations of these linkers, having specific Ar groups, are described in, *e.g.,* U.S. Patent No. 6,825,330.

The detectable label or linker is attached either to the platinum ion *(i.e.,* by direct coordination to a site on the platinum ion) or, alternatively, to a stabilizing substituent attached to the platinum ion *(e.g.,* to a substituent participating in a stabilizing bridge structure). For example, in specific variations in which the platinum complex includes an aliphatic diamine bridge with terminal nitrogens bound to the platinum ion, the detectable label or linker can be attached to one of the terminal nitrogens. When the detectable or linker is attached to the platinum ion, the detectable label or linker has a reactive group that displaces the leaving group from the platinum ion, thereby attaching the detectable label or linker to the platinum ion. In those embodiments comprising the use of a linker, the detectable label, *e.g.,* a fluorescent dye, is typically already attached to the opposite end of the linker, such that the reaction of the linker with the platinum compound results in a platinum labeling complex.

When a linker is to be attached to a terminal nitrogen of an aliphatic diamine (the nitrogen being bound to the platinum ion), the linker can, for example, be first attached to the detectable label *(e.g.,* by reaction of a linker carboxylate with a dye-NHS) and the linker can then be reacted with the aliphatic diamine to generate a labeled aliphatic diamine, which can then be reacted with platinum to generate a platinum labeling complex.

Standard chemical synthetic techniques known to those skilled in the art can be used to make platinum labeling complexes in accordance with the present invention. One suitable approach acceptable for the generation of platinum labeling compounds comprising an ethylenediamine bridge is described in, for example, U.S. Patent No. 5,714,327 to Houthoff et al. In this approach, potassium tetrachloroplatinate is first converted to a tetraiodoplatinate. The tetraiodoplatinate is reacted with ethylenediamine, to generate a Pt(II) ethylenediamine diiodide. The diiodide is transferred to its nitrate derivative through ligand exchange, forming the Pt(II) ethylenediamine dinitrate, which is then reacted with a detectable label *(e.g.,* a fluorescent dye) bearing an activated linker. Similar approaches can be used for other platinum labeling complexes. For example, the ethylenediamine can be substituted with a cycloaliphatic diamine to adapt this method to generate platinum labeling compounds having a cycloaliphatic diamine bridge, such as described in, *e.g.,* U.S. Patent No. 6,825330 to Braman et al. This method will generate monofunctional compounds in which the detectable label is attached to a nitrogen that is not part of the aliphatic diamine stabilizing bridge, meaning that there is room for only one functional leaving group. The synthesis of platinum labeling compounds is also described in, *e.g.,* Heetebrij et al., ChemBioChem 4:573-583, 2003; WO 96/35696; WO 98/15564, and U.S. Patent No. 5,580,990.

Another suitable approach for generating platinum labeling complexes comprising an aliphatic diamine bridge, and which avoids the need for a ligand exchange step, involves directly reacting potassium tetrachloroplatinate (II) with an aliphatic diamine *(e.g.,* ethylene diamine or a cycloaliphatic diamine) bearing a detectable label. The reaction forms a labeled platinum aliphatic diamine dichloride. The aliphatic diamine forms a stabilizing bridge and the two chloride ions serve as leaving groups available for subsequent reaction with a guanine nucleotide. This approach is also described in, *e.g.,* U.S. Patent No. 6,825,330.

Methods of preparing fluorescent dyes or other detectable labels bearing activated groups for reaction with various functional groups *(e.g.,* amines) are known in the art. Table 2 lists a number of reactive groups that are routinely used to react with certain functional groups.

**Table 2.** Selected Reactive Substituents and Corresponding Functional Groups Reactive Therewith.

| Reactive Group(s) | Corresponding Functional Group(s) |
|---|---|
| succinimidyl esters | amines |
| anhydrides | amines, alcohols |
| acyl azides | amines |
| isothiocyanates | amines, thiols, alcohols, phenols |
| sulfonyl chlorides | amines, phenols |
| sulfonyl fluorides | alcohols |
| substituted hydrazines, substituted hydroxylamines | aldehydes, ketones |
| acid halides | amino groups |
| haloacetamides, maleimides | thiols, imidazoles, phenols, amines |
| carobdiimides | carboxyl groups |
| phosphoramidite | alcohol groups |

Linkers or detectable labels bearing reactive groups such as those listed in Table 2 can be prepared using methods generally known in the art. For example, (a) succinimidyl esters can be prepared from molecules having a carboxylic acid substituent using N-hydroxysuccinimide and dicyclohexylcarbodiimide; (b) acid chlorides can be prepared from molecules containing carboxylic acid substituents using oxalyl chlorides or thionyl chloride; (c) isocyanates can be prepared from molecules containing amine groups using phosgene; (d) isothiocyanates can be prepared from molecules having amino substituents using thiophosgene; (e) photoaffinity labels can be incorporated by reaction of an amine-containing molecule and a known photoaffinity label that also contains an amine-reactive group; and (f) maleimido groups can be prepared from an amine-containing dye or other detectable marker and maleic anhydride.

In some embodiments, the labeled hairpin structure is attached to the chromosome-specific sequence with a crosslinker or a spacer). Crosslinkers may be homobifunctional or heterobifunctional. Suitable homobifunctional crosslinkers include, *e.g.,* amine reactive crosslinkers with NHS esters at each end (including, *e.g.,* dithiobis(succinimidylpropionate) (DSP); 3, 3'-dithiobis(sulfosuccinimidylpropionate) (DTSSP); disuccinimidyl suberate (DSS); Bis(sulfosuccinimidyl)suberate (BS3); Ethylene glycolbis(succinimidylsuccinate) (EGS); Ethylene glycolbis(sulfosuccinimidylsuccinate) (SulfoEGS)); amine reactive crosslinkers with imidoesters at both ends (including, *e.g.,* dimethyl adipimidate (DMA); dimethyl pimelimidate (DMP); dimethyl suberimidate (DMS); dimethyl 3,3'-dithiobispropionimidate (DTBP)); sulfhydryl reactive crosslinkers with dithiopyridyl groups at each end (including, *e.g.,* 1,4-di-[3'-(2'-pyridyldithio)propionamdo]butane (DPDPB)); sulfhydryl reactive crosslinkers with maleimide groups at each end (including, *e.g.,* bismaleimidohexane (BMH)); carboxyl reactive crosslinkers with hydrazide groups at each end (including, *e.g.,* adipic acid dihydrazide and carbonhydrazide); multi-group reactive crosslinkers with epoxide groups at each end (including, *e.g.,* 1,2:3,4-diepoxybutane; 1,2:5,6-diepoxyhexane; Bis(2,3-epoxypropyl)ether; 1,4-(butanediol) diglycidyl ether). Suitable heterobifunctional crosslinkers include crosslinkers with an amine reactive end and a sulfhydryl-reactive end (including, *e.g.,* N-Succinimidyl 3-(2-pyridyldithio)propionate (SPDP); long chain SPDP (SPDP); Sulfo-LC-SPDP; Succinimidyloxycarbonyl-α-methyl-α-(2-pyridydithio)toluene (SMPT); Sulfo-LC-SMPT; Succinimidyl-4-(N-maleimidomehyl)cyclohexane (SMCC); Sulfo-SMCC; Succinimidyl 6-((iodoacetyl)amino)hexanoate (SIAX); Succinimidyl 6-(6-(((4-iodoacetyl)amino)hexanoyl)amino)hexanoate (SIAXX)); crosslinkers with a carbonyl-reactive end and a sulfhydryl reactive end (including, *e.g.,* 4-(4-N-Maleimidophenyl)butyric acid hydrazide (MPBH); 4-(N-Maleimidomethyl)cyclohexane-1-carboxyl-hydrazide hydrochloride (M2C2H); 3-(2-Pyridyldithio)propinyl hydrazide (PDPH)); crosslinkers with an amine-reactive end and a photoreactive end (including, *e.g.,* Sulfosuccinimidyl-2-(p-azidosalicylicylamido)ethyl-1,3'-dithiopropionate (SASD); Sulfosuccinimidyl 2-(7-azido-4-methylcoumarin-3-acetamide)ethyl-1,3'- dithiopropionate (SAED)); crosslinkers with a sulfhydryl-reactive end and a photoreactive end (including, *e.g.,* N-[4-p-Azidosalicylamido)butyl]-3'-(2'pyridyldithio)propionamide (APDP)); crosslinkers with a carbonyl-reactive end and a photoreactive end (including, *e.g.,* 4-(p-Azidosalicylamido)butlyamine (ASBA)). Suitable spacers include, 5' ODN modifications such as dNTP's; and amine-reactive spacers such as amino- or sulfo-phosphoramidites including, *e.g.,* butylphosphoramidites, pentylphosphoramidites, hexylphosphoramidites, heptylphosphoramidites, octylphosphoramidites, nonylphosphoramidites, decylphosphoramidites, undecylphosphoramidites, dodecylphosphoramidites, pentadecylphosphoramidites, octadecylphosphoramidites. Other suitable amine-reactive spacers include e.g., activated polyethylene glycol (PEG) such as (monomethoxy)n glycol, wherein n=3-18 unit repeats. Additional suitable crosslinkers and spacers are set forth in Herman. "Bioconjuate Chemistry". Academic Press. New York, NY. 1996.

One skilled in the art can readily attach a detectable label or linker bearing a reactive group to an oligonucleotide *(e.g.,* a chromosome-specific oligonucleotide or a hairpin oligonucleotide). Similarly, one skilled in the art can readily attach a detectable label or linker bearing a reactive group to a platinum complex having a corresponding functional group reactive therewith *(e.g.,* reaction of a linker containing an amine-reactive group with an aliphatic diamine). For example, any of the amine-reactive functional groups noted in Table 1, or any other groups known in the art to react with amines, can be used for the attachment of a linker or detectable label to a amine-bearing platinum complex. As a non-limiting example, one commonly used coupling method for fluorescent dyes involves reaction of a dye-succinimidyl ester with an amino group on the molecule of interest. Succinimidyl ester-dyes are prepared, for example, by reaction of N-hydroxysuccinimide (NHS) with a carboxyl group on the dye. NHS-modified dyes or isothiocyanate-modified dyes are also commercially available *(e.g.,* from Molecular Probes, Eugene OR). If desired, a fluorescent dye or other label may be modified to have a short linker *(e.g.,* an alkyl chain) between the dye and the NHS active group, for example by reacting an NHS dye with 6-amino-n-caproic acid to form a dye-aminocarboxylic acid. This product is then reacted in anhydrous DMF with NHS to form a dye-NHS compound having the short linker between the dye and the active NHS group. It is this dye-NHS compound that is then reacted with a molecule having a amine group *(e.g.,* an aliphatic diamine) to generate a dye-labeled amine.

Attachment of the platinum complexes to one or more G nucleotides within a G-rich nucleic acid sequence can be carried out using methods generally known in the art for labeling of nucleic acids with platinum-based compounds. For example, in typical embodiments, a platinum labeling complex is simply mixed with an aqueous solution of nucleic acid comprising the G-rich nucleic acid sequence, and the platinum compound forms adducts via the N3 or N7 nitrogen atom of guanine nucleotide bases. Excess labeling compound is removed by standard means and the labeled G-rich sequence is ready for use. In certain variations, the G-rich nucleic acid sequence is labeled before attachment to a target-binding element. Alternatively, the G-rich sequence is already attached to the target-binding element during labeling with the platinum complex. Suitable protocols for labeling of nucleic acids, which can be readily adapted for labeling of G-rich nucleic acid sequences, or nucleic acids comprising such G-rich sequences, are described in, *e.g.,* Wiegant et al., Cytogenet. Cell Genet. 87:47-52, 1999; van Belkum et al., BioTechniques 16:148-153, 1994; WO 96/35696; WO 98/15564; and U.S. Patent Nos. 5,580,990, 5,714,327, and 6,825,330. Typically, using these methods, a plurality of G nucleotides within the G-rich nucleic acid sequence will each be linked to a platinum complex to yield a G-rich sequence having multiple detectable labels, or the capacity to be multiply labeled via attachment of the label to the platinum complex.

## V. Methods of Selecting a Chromosome-Specific Probe Sequence

In another aspect of the present invention, a method of selecting a chromosome-specific probe sequence is provided. A chromosome-specific probe sequence identified by such methods can be used to prepare a chromosome-specific oligonucleotide or labeled probe as set forth above.

In some embodiments, the methods comprise:

(a) comparing a repeat sequence of a specific human chromosome *(e.g.,* chromosome 1, 2, 3, 4, 5, 6, 7, 8, 9,10,11,12,13,14,15, 16, 17, 18, 19, 20, 21, 22, the X chromosome or the Y chromosome) with a consensus repeat

sequence of all human chromosomes; and

(b) identifying a nucleic acid sequence consisting of 25-35 contiguous nucleotides within the repeat sequence of the specific human chromosome and having less than 84% identity to the consensus repeat sequence as a potential chromosome-specific probe sequence.

The methods may further comprise:

(c) comparing the chromosome specific probe sequence identified in (b) with all other contiguous nucleotide sequences within the human genome; and

(d) identifying any sequences that have less than 84% identity to another contiguous nucleotide sequence within the human genome as potential chromosome specific probe sequences.

The methods may further comprise:

(e) chemically synthetizing and labeling the chromosome specific probe sequence with a detectable label;

(f) performing in situ hybridization on human metaphases or interphases using the labeled probe sequence of (e);

(g) detecting the signal associated with the label, wherein a chromosome specific probe sequence that produces a signal at least 2 fold higher upon hybridization is selected as a chromosome specific probe.

In some embodiments, the method for selecting a chromosome-specific probe sequence generally includes the following steps:

(a) comparing centromeric repeat sequences of a specific human chromosome (e.g., chromosome 1, 2, 3, 4, 5, 6, 7, 8, 9, 10, 11, 12,13, 14, 15, 16, 17, 18, 19, 20, 21, 22, the X chromosome or the Y chromosome) with a consensus alpha-monomer repeat sequence of centromeric repeat sequences of all human chromosomes, and

(b) identifying a nucleic acid sequence consisting of 25-35 contiguous nucleotides within the specific centromeric repeat sequence and having less than 84% identity to the consensus alpha-monomer repeat sequence, thereby selecting a chromosome-specific probe sequence.

In other embodiments, the method for selecting a chromosome-specific probe sequence generally includes the following steps:

(a) comparing centromeric repeat sequences of a specific human chromosome (e.g., chromosome 1, 2, 3, 4, 5, 6, 7, 8, 9, 10, 11, 12, 13, 14, 15, 16, 17, 18, 19, 20, 21, 22, the X chromosome or the Y chromosome) with alpha-monomer repeat sequences of all human chromosomes, and

(b) identifying a nucleic acid sequence consisting of 25-35 contiguous nucleotides within the specific centromeric repeat sequence and having less than 84% identity to all other alpha-monomer repeat sequences, thereby selecting a chromosome-specific probe sequence.

These methods can further include the step of preparing an oligonucleotide probe comprising the selected chromosome-specific nucleic acid sequence. Preparation of such probes in accordance with the present invention is further discussed herein *(see* Section II, *supra*).

In certain variations of the methods, the method includes identifying a plurality of nucleic acid sequences as in step (b) above, thereby selecting a plurality of chromosome-specific probe sequences. Some such variations further include the following steps:

(i) preparing a plurality of labeled oligonucleotide probes, each probe comprising a detectable label and a different chromosome-specific nucleic acid sequence, each different sequence being one of the selected chromosome-specific sequences selected;

(ii) performing, individually with each of the different oligonucleotide *probes, in situ* hybridization on human metaphases or interphases;

(iii)detecting a signal associated with the label for each of the hybridized probes; and

(iv) selecting one or more of the oligonucleotide probes based on the degree of detected signal.

In yet other variations, the method further includes (c) comparing the nucleotide sequences of the nucleic acid sequences identified in step (a) with all other contiguous nucleotide sequences within the human genome; and (d) discarding any sequences that have more than 84% identity to another contiguous nucleotide sequence within the human genome. Such embodiments can further include the following steps:

(i) preparing a plurality of labeled oligonucleotide probes, each probe comprising a detectable label and a different chromosome-specific nucleic acid sequence, each different sequence being one of the chromosome-specific sequences not discarded in step (d);

(ii) performing, individually with each of the different oligonucleotide probes, *in situ* hybridization on human metaphases or interphases;

(iii)detecting a signal associated with the label for each of the hybridized probes; and

(iv) selecting one or more of the oligonucleotide probes based on the degree of detected signal.

In identifying a nucleic acid sequence, within the specific centromeric repeat sequence and having less than 84% identity to a consensus alpha-monomer repeat sequence and/or to all other alpha-monomer repeat sequences, percent identity can be determined according to sequence alignment and comparison methods well-known and readily available in the art. In this regard, the term percent "identity," in the context of comparing a specific centromeric repeat sequence with a consensus alpha-monomer repeat sequence and/or to all other alpha-monomer repeat sequences, refers to two or more sequences or subsequences that have a specified percentage of nucleotides that are the same *(e.g.,* 60%, 65%, 70%, 75%, 76%, 77%, 78%, 79%, 80%), when compared and aligned for maximum correspondence over a comparison window or designated region as measured using a sequence comparison algorithm or by manual alignment and visual inspection.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities or similarities for the test sequences relative to the reference sequence, based on the program parameters.

Methods of alignment of sequences for comparison are well known in the art. Optimal alignment of sequences for comparison can be conducted, for example, by the local homology algorithm of Smith and Waterman (Adv. Appl. Math. 2:482, 1970), by the homology alignment algorithm of Needleman and Wunsch (J. Mol. Biol. 48:443, 1970), by the search for similarity method of Pearson and Lipman (Proc. Natl. Acad. Sci. USA 85:2444, 1988), by computerized implementations of these algorithms (e.g., GAP, BESTFIT, FASTA, and TFASTA in the Wisconsin Genetics Software Package, Genetics Computer Group, 575 Science Dr., Madison, Wis.), or by manual alignment and visual inspection *(see, e.g.,* Ausubel et al., Current Protocols in Molecular Biology (1995 supplement)). Examples of useful algorithms include, *e.g.,* PILEUP, which uses a simplification of the progressive alignment method of Feng and Doolittle (J. Mol. Evol. 35:351-360, 1987) and which is similar to the method described by Higgins and Sharp (CABIOS 5:151-153, 1989);

An example of a useful algorithms is, PILEUP, which uses a simplification of the progressive alignment method of Feng and Doolittle (J. Mol. Evol. 35:351-360, 1987) and which is similar to the method described by Higgins and Sharp (CABIOS 5:151-153, 1989). Other examples of algorithms that are suitable for determining percent sequence identity are BLAST and BLAST 2.0 algorithms, which are described in Altschul et al. (Nuc. Acids Res. 25:3389-402, 1977), and Altschul et al. (J. Mol. Biol. 215:403-10, 1990), respectively. Software for performing BLAST analyses is publicly available through the National Center for Biotechnology Information website (http://www.ncbi.nlm.nih.gov/).

## VI. Chromosome Detection

Labeled probes as described above are useful in any method comprising a nucleic acid hybridization assay that calls for, or is otherwise amenable to, a chromosome-specific probe comprising the selected detectable label. Advantages of using labeled probes of the present invention include, for example, relatively short hybridization times (5-10 minutes compared to 6-16 hours with genomic derived FISH probes) as well as the signals and/or signal-to-noise ratios achieved.

Accordingly, in another aspect, the present invention provides a method for detecting a specific human chromosome. The method generally includes contacting a preparation of human chromosomes with a labeled, chromosome-specific probe as described herein; incubating the chromosome preparation and the probe under conditions sufficient to allow the probe to hybridize to, if present, a specific human chromosome having a centromeric repeat sequence perfectly complementary to the chromosome-specific sequence of the probe; and detecting the label to detect the specific human chromosome, if present. The method can be, for example, a method for identifying a chromosomal abnormality. In some variations, the chromosome preparation includes a human cell and the hybridization step is performed *in situ* on chromosomes within the cell.

In typical embodiments, the detection method involves fixing a chromosome sample, such as to obtain a chromosome spread. A variety of suitable fixatives are available and include, for example, acid acetone solutions, various aldehyde solutions *(e.g.,* formaldehyde, paraformaldehyde, and glutaraldehyde) and acid alchohol solutions. Examples of specific chromosomal fixatives are discussed, for example, in Trask et al. (Science 230:1401-1402, 1985).

Preferably, the chromosome sample (*e.g.,* a fixed chromosome sample) is treated to make the target DNA more accessible to the probe. For example, treating a chromosomal preparation with an agent or agents that remove proteins from the chromosomes can enhance the ability of probes to access target sites in the chromosomes. Agents useful for performing such removal include certain enzymes (e.g., pronase or proteinase K, trypsin, and pepsin) and mild acid. Deproteinization is performed with an enzyme concentration and for a time period that increases accessibility to allow for hybridization between probe and target sites; the period, however, is sufficiently short to prevent a loss of morphological detail that renders subsequent chromosomal identification difficult. An alternative is to deproteinize chromosomes by mild acid extraction. Thus, certain deproteinization methods involve treating a chromosomal preparation with 0.02-0.2 N HCl followed by high temperature (e.g., 70°C) washes. An optional pretreatment with RNase can be utilized to remove residual RNA from the chromosomal preparation.

Also, treatment to increase accessibility of target DNA to probe typically includes denaturing chromosomes. Various denaturation conditions can be utilized. Certain denaturation methods include incubation in the presence of high pH, low pH, high temperature and/or various organic solvents (e.g., formamide, tetraalkylammonium halides). Denaturants are usually removed prior to contacting chromosomes with the probes using established washing procedures. For example, when formamide is used as a denaturing agent, common washes involve a series of ice-cold ethanol rinses at 70%, 80%, and 90% ethanol. Denaturation can also be performed after putting the probe on the slide and increasing the temperature (75-85°C) for 5 min before the hybridization step.

Once a chromosomal preparation is contacted with a chromosome-specific probe, hybridization is conducted under conditions and for a time period sufficient for the probe to hybridize with the complementary sequences in the chromosomes. Conditions suitable for specific binding of an oligonucleotide probe to a corresponding target are generally known in the art. (*See, e.g.,* Sambrook and Russell, *supra;* Ausubel *et al., supra. See also* Tijssen, Laboratory Techniques in Biochemistry and Molecular Biology, Vol. 24: Hybridization with Nucleic Acid Probes (Elsevier, NY 1993)) Optimal hybridization conditions typically depend upon several factors such as salt concentration, incubation time, and concentration, composition, and length of the probes as will be appreciated by those of ordinary skill in the art. Based on these and other known factors, suitable binding conditions are readily determined and, if necessary, optimized for use in accordance with the present methods.

Hybridization is carried out under stringent conditions that allow formation of stable and specific binding of substantially complementary strands of nucleic acid and any washing conditions that remove non-specific binding of the probe. Generally, stringency occurs within a range from about 5° C below the melting temperature ($T_m$) of the probe to about 20° C-25° C below the $T_m$. Stringency can be increased or decreased to specifically detect target nucleic acids having 100% complementarity or to also detect related nucleotide sequences having less than 100% complementarity. In certain methods, very stringent conditions are selected to be equal to the $T_m$ for a particular probe. Factors such as the length and nature (DNA, RNA, base composition) of the sequence, nature of the target (DNA, RNA, base composition, presence in solution or immobilization) and the concentration of the salts and other components *(e.g.,* the presence or absence of formamide, dextran sulfate and/or polyethylene glycol) are considered and the hybridization solution can be varied to generate conditions of either low, medium, or high stringency. Washing conditions typically range from room temperature to 60° C.

For example, high stringency conditions can include, *e.g.,* 2xSSC, 30% formamide, 10% Dextran Sulfate at 37°C for the hybridization, followed by 3 washes at 45°C in 30% formamide 2xSSC, 5 washes in 2xSSC at 45°C and one wash at 45°C in 1xPBD (Na2HPO4 0.1 M, $NaH_2PO_4$ 0.6 mM, $NaN_3$ 0,003%, Nonidet P40 0,05%). Other suitable high stringency conditions include 6×NaCl/sodium citrate (SSC) at about 45 °C for a hybridization step, followed by a wash of 2xSSC at 50 °C; or hybridization at 42 °C in 5×SSC, 20 mM $NaPO_4$, pH 6.8, 50% formamide, followed by a wash of 0.2×SSC at 42 °C. Other suitable high stringency conditions include 0.5xSSC, 0.1%SDS at 50°C for 2 minutes These conditions can be varied based on nucleotide base composition and length and circumstances of use, either empirically or based on formulas for determining such variation *(see, e.g.,* Sambrook *et al., supra;* Ausubel *et al., supra*). Depending on base composition, source, and concentration of target nucleic acid, other conditions of stringency can be

used, including, for example, low stringency conditions *(e.g.,* 4-6×SSC/0.1-0.5% w/v SDS at 37-45 °C for 2-3 hours) or medium stringency conditions *(e.g.,* 1-4×SSC/0.25-0.5% w/v SDS at 45 °C for 2-3 hours).

In general, there is a tradeoff between hybridization specificity (stringency) and signal intensity. In certain embodiments, the hybridized sample can be washed at successively higher stringency solutions and read between each wash. Analysis of the data sets thereby produced reveals a wash stringency above which the hybridization pattern is not appreciably altered and which provides adequate signal for the particular probes of interest.

Certain differences in the conditions used for hybridization and detection can arise from differences in the detectable label used. For example, when biotin-labeled probes are used, polyvinylpyrrolidone, a constituent of Denhardt's solution, is omitted from the hybridization solution. As another simple example, when the probe is labeled with a fluorescent label, the probe is generally protected from bright light. These differences and others are known to those skilled in the use of various detectable labels that can be attached to a probe in accordance with the present invention.

Once probes have annealed to their complementary sequences, and any unbound probes washed away as needed, a target chromosome can be detected by detecting the presence of a hybridization complex comprising the labeled probe using any method suitable according to the type of detectable label on the probe. As noted above in Section I, labels can be either direct or indirect. Typical direct labels include fluorophores such as, *e.g.,* fluorescein, rhodamine, Texas Red, phycoerythrin, phthalocyanine dyes *(e.g.,* Cy3 or Cy5), Alexa 532, Alexa 546, Alexa 568, and Alexa 594. An indirect process utilizes a binding partner interaction for detection. In these variations, the probe is generally labeled with a molecule that has an affinity for a secondary agent. For example, biotin and haptens such as, *e.g.,* digoxigenin (DIG), DNP, or fluorescein are typical labels which can be detected via an interaction with streptavidin (in the case of biotin) or an antibody as the secondary agent. Following binding of probe and target, the target-probe complex can be detected by using, *e.g.,* directly labeled streptavidin or antibody. Alternatively, unlabeled secondary agents can be used with a directly labeled "tertiary" agent that specifically binds to the secondary agent *(e.g.,* unlabeled anti-DIG antibody can be used, which can be detected with a labeled second antibody specific for the species and class of the primary antibody). The label for the secondary agent is typically a non-isotopic label, although radioisotopic labels can also be used. Typical non-isotopic labels include, *e.g.,* enzymes and fluorophores, which can be conjugated to the secondary or tertiary agent. Enzymes commonly used in DNA diagnostics include, for example, horseradish peroxidase and alkaline phosphatase.

Detection of the probe label can be accomplished using any approach suitable for the particular label. For example, fluorophore labels can be detected using any suitable means known in the art for detecting the emission wavelength of the particular fluorophore used. Typical methods for detecting fluorescent signals include, *e.g.,* spectrofluorimetry, epifluorescence microscopy, confocal microscopy, and flow cytometry analysis. Fluorescent labels is generally preferred for detection of low levels of target because they provide a very strong signal with low background. Also, it is optically detectable at high resolution and sensitivity through a quick scanning procedure, and different hybridization probes having fluorophores with different emission wavelengths *(e.g.,* fluorescein and rhodamine) can be used for a single sample.

In addition, with enzyme labels, detection can be, for example, by color or dye deposition *(e.g.,* p-nitrophenyl phosphate or 5-bromo-4-chloro-3-indolyl phosphate/nitroblue tetrazolium for alkaline phosphatase and 3,3'-diaminobenzidine-$NiCl_2$ for horseradish peroxidase), fluorescence *(e.g.,* 4-methyl umbelliferyl phosphate for alkaline phosphatase) or chemiluminescence (e.g., the alkaline phosphatase dioxetane substrates LumiPhos 530 from Lumigen Inc., Detroit Mich. or AMPPD and CSPD from Tropix, Inc.). Chemiluminescent detection can be carried out with X-ray or Polaroid film or by using single photon counting luminometers. This is a typical detection format for alkaline phosphatase labeled probes.

In certain embodiments of the method, a population of two or more probes is used. A population of probes can be homogeneous with respect to the chromosome-specific nucleic acid sequences. Alternatively, a population of probes can comprise two or more probes with different chromosome-specific sequences. In certain embodiments, a large population of different probes is used. In typical embodiments in which two or more different probes *(i.e.,* probes with different chromosome-specific sequences) are used, the different probes have different detectable labels. Through use of different probes labeled with distinguishable markers, such as spectrally distinguishable fluorophores, combinations of probes can be applied to a chromosomal preparation at the same time in order to examine the presence or absence of two or more specific chromosomes. Standard probing and washing conditions can be used with adjustments made for differing detectable markers.

In the particular case of *fluorescent in situ* hybridization (FISH) procedures, which utilize fluorescent probes, a variety of different optical analyses can be utilized to detect hybridization complexes. Spectral detection methods are discussed, for example, in U.S. Patent No. 5,719,024; Schroeck et al. (Science 273:494-497, 1996); and Speicher et al. (Nature Genetics 12:368-375, 1996). Further guidance regarding general FISH procedures are discussed, for example, in Gall and Pardue (Methods in Enzymology 21:470-480, 1981); Henderson (International Review of Cytology 76:1-46, 1982); and Angerer et al. in Genetic Engineering: Principles and Methods (Setlow and Hollaender eds., Plenum Press, New York, 1985).

In some embodiments, detection of the probe is performed in the absence of digital enhancement or integration. Alternatively, digital enhancement or integration is used to detect a signal.

For example, in certain variations of the method, detection of the label includes the use of microscopic imaging using a CCD camera mounted onto the eyepiece tube of a microscope *(e.g.,* a binocular, monocular, or stereo microscope). In some embodiments, detection of the label is accomplished using image scanning microscopy. For example, recent advances in computerized image scanning microscopy have significantly increased the ability to detect rare cells using fluorescence microscopy, permitting detection of 1 positive cell in an environment of ~$6x10^5$ negative cells *(see, e.g.,* Mehes et al., Cytometry 42:357-362, 2000). Advanced image scanning software has been developed that can not only detect multiple colors but also fused or co-localized signals useful for, *e.g.,* detection of translocations on the DNA level (MetaSystems Group, Inc.) Scanning speed typically depends on the number of parameters utilized for reliable detection of single positive cells. Image scanning also allows for images of the cells scored positive to be manually examined for confirmation. Advanced image scanning software for automated, slide-based analysis has been developed that can not only detect multiple colors but also fused or co-localized signals useful for, *e.g.,* detection of translocations on the DNA level (Meta System Group, Inc.) Scanning speed typically depends on the number of parameters utilized for reliable detection of single positive cells. Automated slide-based scanning systems are particularly amenable to high throughput assays.

In one exemplary embodiment comprising the use of scanning slide microscopy, a MetaCyte Automated Bio-Imaging System (Meta System Group, Inc.) is used. This system consists of the following components: 1) Carl Zeiss Axio Plan 2 MOT fluorescence microscope, 2) scanning 8-position stage, 3) PC Pentium III Processor, 4) Jai camera, 5) camera interface, 6) stage control, 7) trackball and mouse, and 8) printer. The focus analysis begins with a slide set-up loaded onto the microscope. The slide is scanned as the stage is moved and the image is captured. Following scanning of the entire slide, a gallery is created. Based on the criterion set up for positive or negative, the image analysis either results in a positive or negative signal. If negative, the slide is rescanned for rare event analyses. If positive, there is a filter change for the appropriate fluorescent signal and 5-7 planes are captured and analyzed. There is walk away/overnight operation for 8 slides (standard or 100 slides with optional tray changer). Adaptive detection algorithms and automatic exposure control function compensate for non-uniform staining conditions. Several markers can be detected simultaneously. The standard light source covers a wide spectrum from UV to IR. Scanning speed up to 1,000 cells per second can be used for rare cell detection if cellular fluorescent intensity allows detection in 1/1,000 sec. For strong signals, a lower magnification can be used to increase scanning speed.

### VII. Kits for Probe Preparation, Detection Assays, or Primer Extension

In another aspect, a kit is provided for preparing a labeled probe of the present invention. Typically, the kit is compartmentalized for ease of use and contains at least one first container providing an oligonucleotide comprising a chromosome-specific sequence as described herein.

In some embodiments of the kit providing a chromosome-specific oligonucleotide, the first container provides an oligonucleotide comprising a chromosome-specific sequence and a G-rich sequence as described herein, and the kit further contains a second container providing a platinum complex, where the platinum complex includes (a) a first leaving group, characterized by its ability to be replaced by a G nucleotide; and optionally includes (b) a group selected from the following:

(i) a detectable label

(ii) a linker attached to the detectable label;

(iii)a linker capable of attachment to the detectable label; and

(iv)a second leaving group, characterized by its ability to be replaced by at least one of (i), (ii), and (iii) above.

In certain variations in which the platinum complex includes the linker capable of attachment to the detectable label, the kit further comprises a third container providing the detectable label. In other variations in which the platinum complex includes the second leaving group, the kit further comprises a third container providing either the detectable label; the linker attached to the detectable label; or the linker capable of attachment to the detectable label.

In still another aspect, a kit is provided for utilizing a labeled probe of the present invention in detection of a specific human chromosome in a sample. Typically, the kit is compartmentalized for ease of use and contains at least one first container providing a labeled probe as described herein. In some embodiments, the kit provides a plurality of different labeled probes, either mixed in the same container, thereby providing a probe cocktail composition, or in separate containers, which can be used, *e.g.,* for providing a probe cocktail composition. In such embodiments providing a plurality

of different labeled probes, each of the different probes typically has a different chromosome-specific sequence and a different detectable label that is distinguishable from each of the other detectable labels. For example, each detectable label can be a fluorophore having a spectrally distinguishable emission wavelength. Suitable fluorophores for use in such variations of the kit include, e.g., Alexa 488 (excitation maximum at 492 nm and emission maximum at 520 nm) and Alexa 546 (excitation maximum at 555 nm and emission maximum at 570 nm)).

Additional containers providing one or more reagent(s) for detecting the labeled probe can also be included in the kit providing a labeled probe. Such additional containers can include any reagents or other elements recognized by the skilled artisan for use in a detection assay corresponding to the type of label on the probe. In some variations, for example, the probe provided in the first container comprises an indirect label (e.g., biotin) and the kit further includes at least one second container providing a secondary agent for detecting the indirect label *(e.g.,* a container providing streptavidin labeled with a fluorophore).

**EXAMPLES**

As indicated previously, probes as described herein are useful in any method that calls for, or is otherwise amenable to, probe comprising the selected detectable label. The following examples are offered to illustrate a few specific applications of the probes and related methods, but not to limit the claimed invention.

<u>Example 1: Selection of Chromosome Specific Sequences</u>

Nearly 25% of the human genome consists of repetitive sequences. (Miklos, *Molecular Evolutionary Genetics* Plenum Publishing Corp.:241-321, 1985.) Some of these repeats, such as Alu sequences, are highly represented over the entire genome, while others, such as the $\alpha$-satellite repeats and the telomere repeats, are restricted to the centromeres and telomeres, respectively. Additional repetitive DNA families include satellite 1, satellite 2, satellite 3 and the $\beta$-satellite. This example describes selection of probes specific for three specific chromosomes: chromosomes 2, 4, and the Y chromosome.

*1. Chromosome Y Probes*

[The human chromosome Y contains a 3.4 Kb satellite 3 repeat termed DYZ1 *(*Nakagome et al., Cytogenet Cell Genet 56:74-7, 1991) localized in the heterochromatic block at band Yq12 and present at 500 to 3000 copies. This satellite 3 DNA, while containing the TTCCA pentamer, also contains short sequence elements that are highly specific for the Y chromosome (see Fowler et al., Nucleic Acids Res 15:3929, 1987). Throughout this repeat, large numbers of the TTCCA satellite 3 pentamer sequences are interspersed among individual Y-chromosome specific sequence elements of varying length *(see* Nakahori et al., Nucleic Acids Res 14:7569-80, 1986; Weier et al., J Histochem Cytochem 38:421-6, 1990; Nakagome et al., Cytogenet Cell Genet 56:74-7, 1991). The presence of these discrete Y-specific sequences within the satellite 3 repeat permits generation of specific synthetic ODN hybridization probes.

Ideally, for optimal hybridization, synthetic ODN Y-probes are underrepresented for the TTCCA pentamer and consist primarily of sequences comprised of approximately 50% CG-bases. 30mer probes targeting the satellite 3 repeat were designed. To identify the regions that have less than 84% homology with the satellite 3 repeat pentamer $(TTCCA)_n$ The alignment was performed using the bioinformatics tool available at NCBI web site named bl2seq *(*Tatusova et al., FEMS Microbiol Lett 174:247-50, 1999). To exclude all probes that present any homology with other repeated regions of the human genome, all possible 30mer oligonucleotides, available in homology free regions were compared to the whole genome database (NCBI) using the Basic Local Alignment Tool (BLAST) (Altschul et al., J. Mol. Biol. 215:403-10, 1990), publicly available at NCBI web site. More particularly, the sequences were compared to the non redundant genomic database (nr) with no filter. The 6 probes retained are described in Table 3.

**Table 3.** Y Chromosome-Specific Nucleic Acid Sequences for Oligonucleotide Probes.

| SEQ ID NO: | Sequence | Name | GC content |
|---|---|---|---|
| 1 | 5'-ccagtcgaatccattcgagtacatacc | Y1 | 48% |
| 2 | 5'-ccttttgaatccattccattggagtcc | Y2 | 33% |
| 3 | 5'-attcattgcattccgtttcatgaaattcga | Y3 | 43% |
| 4 | 5'-ctgcatacaatttcactccattcgttccca | Y4 | 47% |
| 5 | 5'-tccattggagtcaattcctttcgacaccca | Y5 | 47% |

(continued)

| SEQ ID NO: | Sequence | Name | GC content |
|---|---|---|---|
| 6 | 5'-ttgatcctattttattaaattgcattctat | Y6 | 58% |

*2. Chromosome 4 Probes*

The pericentromeric region of the human 4 chromosome is characterized by 3.2 Kb tandemly repeated DNA consisting entirely of α-satellite DNA comprised of dimeric periodicity of α-satellite monomers (171 bp) D1-D2 arranged in imperfect, direct repeats. *(See* Waye et al., Proc Natl Acad Sci USA 86:6250-4, 1989). The repeated 3.2 Kb sequence is present thousands of times in the centromeric heterochromatin region of human 4 chromosome (Mashkova et al., Gene 140 (2), 211-217, 1994). The imperfect, direct repeats can conveniently be used to design probes that are specific for the 4 chromosome α-satellite repeats. To avoid any cross hybridization of our chromosome 4 probes to other chromosome α-satellite repeats, the probes were designed using the sequence of the pYAM7-62 clone that have lower homology with the consensus α-monomer sequence. To identify the regions that have less than 84% homology with the α-monomer, the consensus α-monomer sequence was aligned with the 685bp chromosome 4 centromere sequence. The alignment was performed using the bioinformatics tool available at NCBI web site named bl2seq *(*Tatusova et al., FEMS Microbiol Lett 174:247-50, 1999). Then, in order to exclude all probes that present any homology with other repeated regions of the human genome, all possible 30mer oligonucleotides, available in homology free regions were tested by BLAST on the non-redundant database. Retained chromosome 4 ODN probes are described in Table 4.

**Table 4.** Chromosome 4-Specific Nucleic Acid Sequences for Oligonucleotide Probes.

| SEQ ID NO: | Name | Sequence |
|---|---|---|
| 14 | 4.3.2 | TCTTTGTGTTGTGTGTACTCATGTAACAGT |
| 15 | 4.6.2 | TTTCTGCCCTACCTGGAAGCGGACATTTCG |
| 16 | 4.7.5 | GGTTATCTTCATATAAAATCCAGACAGGAG |
| 17 | 4.10.2 | CGGCACTACCTGGAAGTGGATATTTCGAGC |
| 18 | 4.18.7 | TCTGCACTACCTGGAAGAGGCCATTTCGAG |
| 19 | 4.22.10 | CCTACGGGGAGAAAGGAAATATCTTCAAAT |

*3. Chromosome 2 Probes*

The pericentromeric region of the human 2 chromosome is characterized by 1.05-2.9 Kb tandemly repeated DNA consisting entirely of α-satellite DNA comprised of trimers, tetramers and pentamers of α-satellite monomers (171 bp) arranged in imperfect, direct repeats. (Haaf and Willard, Genomics 13, 122-128, 1992). The repeated 3.2 Kb sequence is present thousands of times in the centromeric heterochromatin region of human chromosome2 *(*Rocchi et al., Genomics 8, 705-709, 1990*)*. The imperfect, direct repeats can conveniently be used to design probes that are specific for the chromosome 2 α-satellite repeats. To avoid any cross hybridization of our chromosome 2 probes to other chromosome α-satellite repeats, the probes were designed using the sequence of the pBS4D clone that have lower homology with the consensus α-monomer sequence. To identify the regions that have less than 84% homology with the α-monomer, the consensus α-monomer sequence was aligned with the 685bp chromosome 4 centromere sequence. The alignment was performed using the bioinformatics tool available at NCBI web site named bl2seq *(*Tatusova et al., FEMS Microbiol Lett 174:247-50, 1999). Then, in order to exclude all probes that present any homology with other repeated regions of the human genome, all possible 30mer oligonucleotides, available in homology free regions were tested by BLAST on the non-redundant database. Retained chromosome 2 ODN probes are described in Table 5.

**Table 5.** Chromosome 2-Specific Nucleic Acid Sequences for Oligonucleotide Probes.

| SEQ ID NO: | Name | Sequence |
|---|---|---|
| 7 | 2.1.1 | GTGCGCCCTCAACTAACAGTGTTGAAGCTT |
| 8 | 2.2.2 | GAAACGGGATTGTCTTCATATAAACTCTAG |
| 9 | 2.5.1 | GTATCTTCCAATAAAAGCTAGATAGAAGca |

(continued)

| SEQ ID NO: | Name | Sequence |
|---|---|---|
| 10 | 2.6.1 | atgTCAGAAACTTTITCATGATGTATCTAC |
| 11 | 2.7.3 | TATGTGTGATGTGCGCCCTCAACTAAGAGT |
| 12 | 2.8.4 | TCTCAGAAGCTTCATTGGGATGTTTCAATT |
| 13 | 2.10.1 | GGAATACGGTGATAAAGGAAATATCTTCCA |

Example 2: Characterization of Directly Labeled Chromosome-Specific Probes

Two probe cocktails containing four ODNs targeting chromosome Y (Y2, Y3, Y4, and Y5) either directly 5' end-labeled or G-tail-labeled with the fluorophore Alexa 546 were generated and used as probes. Table 6 sets forth the data demonstrating that there were no significant differences between the two probe cocktails.

**Table 6.** Results of 5' End-Labeled vs. G-Tail-Labeled Comparison.

| | End labeled | G-tail labeled |
|---|---|---|
| Signal/noise (mean) | 2.45 | 2.32 |
| STD error of the mean (95%) | 0.07 | 0.05 |

Additional experiments using individual directly end-labeled or G-tailed labeled probes were conducted to determine which labeling method leads to greater detection sensitivity. Table 7 sets forth data demonstrating that the end-labeled probes generated a higher signal to noise ratio.

**Table 7.** Detection Sensitivity Results Comparing End-Labeled vs. G-Tail-Lableled Probes.

| | End labeled | G-tail labeled |
|---|---|---|
| DY549/KR547 | 3.7±0.06 | 2.8±0.05 |
| DY590/KR590 | 2.5±0.07 | 1.8±0.07 |
| DY490/KR495 | 2.0±0.04 | 1.6±0.03 |

One advantage of using end-labeled oligonucleotides is that any fluorophore can be used for end labeling. The end-labeled oligonucleotides can also be labeled with a terminal amino-group at their 5' end that can subsequently be labeled with any fluor NHS-ester. For G-tail labeling, only fluors that have been adapted to the ULS chemistry can be used.

The oligonucleotide probes described herein have several advantages including a dramatically shortened hybridization time for *in situ* hybridization and elimination of the need for a sample pretreatment step. The dramatically reduced hybridizations times may be related to the length of the probes, *i.e.,* the shorter probes may be able to quickly penetrate the cell and hybridize with their target. Currently available commercial probes targeting the same repeat sequences require a 2-8 hours hybridization time, but the oligonucleotide probes described herein show a higher signal to noise ratio following a shorter incubation time (e.g., as little as 1 minute). Omission of the pretreatment step resulted in only small decrease of the signal to noise ratio (~0.5) demonstrating that these treatments were not needed for the probes described herein.

Example 3: Hairpin Probes to Detect Specific Chromosomes

This example compares hairpin probes comprising the exemplary hairpin structures set forth in Figure 1 which vary by number and position of amino-modified dC and dT nucleotides which provide a amine group for interaction with a detectable label *(e.g.,* a fluorophore. The hairpin structures comprise one amino group every three bases, every six bases, or less. One of the hairpin structures comprises amino groups on only on strand of the stem structure. Hairpin probes comprising different detectable labels can be used to simultaneously detect multiple targets in the same sample without the need for post-hybridization detection.

The 4 ODNs selected for chromosome Y were synthesized with the hairpin structure D (Figure 1) on the 5' end. The oligonucleotides were labeled with DY549-NHS ester (Dyomics, Jena, Germany). After labeling probes were purified by PAGE and the higher bands containing the ODNs with the highest number of dye molecules were purified. The molar

ratio of labeling showed that these probes contained 4 dyes per oligonucleotide and were combined together and hybridized on cytogenetic slides from a chromosomally normal human male. Signal to noise ratio was calculated as previously described and had a mean of 8.5±0.5 with a maximum value of 16.3. These results compared with the same probes end labeled show that the signal to noise ratio increases with the number of dyes per probe and stresses the high intensity of the signals obtained with this labeling.

Materials and Methods

*1. Synthesis of Labeled Hairpin Probes*

Chromosome-specific oligonucleotide sequences were selected as set forth in Example 1 above and chromosome-specific ODN probes comprising hairpin sequences were synthesized. Post-synthesis, the ODN probes were incubated with fluorophore-conjugated n-hydroxysuccinimide (NHS) esters, which form covalent amide bonds only with the reactive amine groups of the ODN, resulting in an ODN probe with multiple labels restricted to the non-hybridizing stem-loop structure. A wide variety of different fluorophore-NHS ester formulations are commercially available, enabling production of directly labeled ODN probes with diverse fluorophores. After synthesis, any number of different fluorophore-NHS esters can be linked to the amine groups using standard coupling chemistry, eliminating the need for post-hybridization detection.

After labeling ODN hairpin probe constructs with the fluorophore Alexa 488, they were hybridized in solution to complementary sequence ODNs conjugated to microspheres (capture probe), prepared by reacting a molar excess of complementary ODN, synthesized with a single biotin at the 3'-end, with 6 micron Streptavidin coated microspheres (Polysciences, Inc., Warrington, PA), for 3hours at room temperature. Unbound ODN were removed by 4 washes in 2xSSC, followed by recovery of the microspheres by centrifugation at 1500 RPM for 5min. Equal molar amounts of different labeling reactions were hybridized to aliquots of the capture probe in 40% formamide/2xSSC for 2hours at 37°C, followed by washes at 37°C, 15min each, in 1xSSC, and 0.2xSSC. Microsphere recovery was done by centrifugation as above. Microspheres with bound labeled probe were resuspended in PBS, and analyzed by flow cytometry using a FACScan cytometer (BD BioSciences, Inc., Franklin Lakes, NJ). Microspheres were identified by forward and side scatter characteristics and gated accordingly. Alexa 488 fluorescence within the gated region was detected using the FL1 channel. Results were plotted as Maximum Fluorescence Intensity (MFI) versus labeling reaction.

*2. In Situ Hybridization*

Cellular DNA was denatured by incubating in 70% formamide/2XSSC for 2min at 72°C. Slides were then immediately placed in cold 70% ethanol for 2min and dehydration was continued in 80%, 90% and 100% ethanol for 2min each and then air dried. Probe solution (5-10μl) were placed on each spot or spread and covered with a ParafilmTM coverslip. Hybridization was done at 37°C using several time points from 15min to 17hours (overnight). Probe solution contained 20-50% formamide, 2XSSC, 10% Dextran Sulfate as well as 0.02- 0.1mM labeled probe and 1μg of sonicated salmon sperm DNA. For each probe, optimal probe and formamide concentration was determined empirically. After hybridization, slides were washed at 45°C in 20-50% formamide/2xSSC for 3min 3 times followed by 5 washes with 2xSSC for 2min and one wash with 1xPhosphate Buffer with Detergent (1xPBD 100mM Na2HPO4, 6mM NaH2PO4, 0.025% Nonidet P40 at pH 8). Finally, the slides were kept in a 1xPBD bath at room temperature. Direct fluorescence labeled probes were visualized using epifluorescence microscopy after mounting and DNA counterstaining with phenylenediamine antifade solution containing DAPI 200 ng/mL.

*3. Fluorescence Microscopy and Image Acquisition*

Fluorescence microscopy analysis and digital image capture were performed using a Nikon Eclipse E600 microscope (Nikon Instruments, Melville, NY) equipped with a cooled CCD camera (Photometrics Ltd., Tucson, AZ). Images were captured using IP Lab Spectrum software (Scanalytics, Inc., Fairfax, VA), and final images were merged using Photoshop™ software (Adobe Systems Inc., San Jose, CA).

Flow cytometry was used to accurately measure fluorescence intensity. After synthesizing the same ODN with the four different structures and labeling with Alexa488 NHS ester under identical conditions, the AminoLoop™-ODNs were hybridized to complementary sequence ODNs bound to 6μm microspheres prepared by incubating 5'-biotinylated ODNs to Streptavidin coated microspheres. The microsphere/ODN complex was analyzed by flow cytometry as described in Materials and Methods. A control ODN, synthesized with a single 5'-fluorescein, served as a control for fluorescence signal. The construct exhibiting the highest fluorescence intensity was used for all further ODN synthesis reactions and labeling.

*4. Calculating Analytical Specificity and Sensitivity Sample Size.*

According to the Standards and Guidelines for Clinical Genetic Laboratories of the ACMG (November 2003), analytical specificity and analytical sensitivity of a FISH probe are assessed by analyzing 200 metaphase cells from 5 chromosomally normal individuals, yielding analytical sensitivity and specificity values ≥98% at 95% confidence. These guidelines were established for conventional diagnostic FISH applications, where a statistically large sample population, in terms of number of cells available for analysis, exists. With recent focus on more restrictive applications of FISH, such as in PGD, where only a single cell is available for analysis, we established a 99% analytical sensitivity and specificity value, with 95% confidence.

Confidence interval for analytical specificity and sensitivity is designated ($p_{low}$,1), where the upper limit of our interval is 100% specificity or sensitivity (1) and the lower limit (plow) is 99%, with a 95% confidence interval (significance $\alpha$=0.05).

$$n = \frac{\ln(\alpha)}{\ln(p_{low})} \qquad n = \frac{\ln(0.05)}{\ln(0.99)} = 298.07$$

*5. Calculating Analytical Specificity and Sensitivity*

Chromosomes were identified by reverse DAPI banding, followed by physical localization of the FISH signal. Valid signal was indicated by the presence of characteristic double signal (covering both chromatids) localized at the correct locus. Inappropriate localization indicates a lack of analytical specificity. Analytical specificity is calculated as follows:

$$Analytical_Specitificity(\%) = \frac{Number_of_cells_with_signal_only_at_correct_locus}{Total_number_of_cells_analyzed_with_FISH_signals} \times 100$$

Analytical Sensitivity is defined as the percentage of cells in a sample with the correct number of hybridization signals at the targeted locus, calculated as follows:

$$Analytical_Sensitivity(\%) = \frac{Number_of_cells_with_\text{expected}_number_of_specific_signals}{Total_number_of_cells_analyzed} \times 100$$

*6. Calculating Signal Intensity*

Images from 30 interphase nuclei (minimal number required for statistical analysis) were analyzed to determine signal to noise ratio. Using IPLabSpectrum software, FISH images acquired under identical conditions were separated into cell signal and noise regions by thresholding the gray level to differentiate between signal and cell nucleus. For each cell, the gray level mean (sum of measured cell pixel gray levels divided by the cell pixel area) was calculated. Signal is defined as the region containing pixel intensities > 2 S.D. of the cell mean pixel value. Mean signal value was then calculated as the sum of measured signal pixel gray levels divided by the signal pixel area. Signal to noise ratio was then defined as the signal mean pixel value divided by the background mean pixel value.

To assess performance of labeled hairpin probes, FISH results were compared to those obtained using commercially available FISH probes. To quantitatively assess performance of labeled hairpin probes, analytical specificity, analytical sensitivity, and signal to noise ratio were measured and compared to results obtained using commercial probe preparations targeting the same genetic loci. Based on established criteria described in the Guidelines and Standards of the American College of Medical Genetics (ACMG), commercial FISH probes must exhibit analytical specificity and sensitivity greater than or equal to 98%.

Individual probes were tested by performing FISH on metaphase chromosome preparations from chromosomally normal males at three different hybridization stringency conditions, resulting from use of formamide denaturant at 20%, 35% and 50%. Note that chromosomal normalcy was previously confirmed by conventional karyotype analysis. 60 metaphase chromosome fields from each of 5 individuals were analyzed using the criteria described above; analytical specificity, analytical sensitivity, and signal intensity, for each ODN probe at each stringency. Probes or probe cocktails which generate ≥98% analytical specificity to the targeted region, ≥98% hybridization efficiency, and a signal/noise ratio ≥ signal/noise ratio of the commercial probe were selected.

Example 4: Use of G-tailed labeled oligonucleotide probes in Preimplantation Genetic Diagnosis

A. Introduction

*1. Current PGD methods*

PGD is performed on day 3 after IVF when a blastomere is biopsied, fixed and prepared for FISH analysis. The availability of only a single cell, and the requirement that the embryo has to be replaced in the patient on day 4, represent significant constraints *(see* Munne et al., Reprod Biomed Online 7:91-7, 2003.) For ploidy analysis, the biopsied cell is analyzed by FISH, at a minimum, with probes specific for chromosomes: X, Y, 13, 15, 16, 18,21 and 22. A maximum of three rounds of FISH can be performed on a fixed cell without compromising the reliability of analysis, and only probes labeled with one of 5 fluorochromes in the visible spectrum are currently used. Thus, in order to analyze 8 chromosomes, two rounds of FISH are currently performed. All commercially available probes are large plasmids or bacterial artificial chromosome (BAC) genomic clones containing either the specific part of the repeat DNA sequence of a certain chromosome, or a unique sequence specific to a certain region localized near the centromere. Present probes require from 8 to 16 hour hybridization. Spot counting in the interphase nuclei of the biopsied determines the ploidy of the embryo, allowing classification of the embryo into one of five categories *(see* Munne *et al., supra*):

1. Normal in which two copies of all the chromosomes are detected.

2. Polyploid in which three or more copies of all the chromosomes are detected.

3. Haploid in which one or fewer copies of all the chromosomes are detected.

4. Aneuploid in which one or two chromosomes with an abnormal number of copies are detected.

5. Complex abnormal in which three or more chromosomes with an abnormal number of copies are detected.

Only embryos classified as normal, of course, are replaced in the patients. As described, the reliability of PGD is entirely dependent on the accuracy of FISH analysis on a single cell. Detection, sensitivity and probe specificity are the primary requirements for successful FISH. *(See* Pinkel et al., Proc Natl Acad Sci USA 85:9138-42, 1988). For ease of use, direct fluorescent labeled probes are preferred, since they do not require additional steps or reagents to visualize the target sequence. However, they are not as sensitive as probes labeled with haptens, such as biotin or digoxigenin.

*2. General Considerations for Use of ODNprobes in PGD*

A significant benefit of ODN probes is their small size and low complexity, which is a measure of the possible number of sequence combinations contained in a probe preparation. These traits result in faster hybridization kinetics (~5min.) compared to high complexity probes, which require 6-16 hours for hybridization. Targeting repetitive DNA eliminates some sensitivity issues, particularly if the repeated sequence unit is present in thousands of copies, such as the satellite 3 region of the human Y chromosome *(see* Nakagome et al., Cytogenet Cell Genet 56:74-7, 1991) or the centromeric $\alpha$-satellite repeats at the locus DXZ1 *(see* Waye et al., Proc Natl Acad Sci USA 86:6250-4, 1989).

*3. Current ODN labeling strategies*

A variety of synthetic and enzymatic methods are currently used to label ODN probes non-radioactively. These methods include labeling one or both ends of the ODN, labeling nucleotides internal to the ODN, enzymatically adding a plurality of labels to one end (tailing), or incorporating branched label structures *(see* Brakel, End Labeled Nucleotide Probe, 1992; van Gijlswijk et al., JHistochem Cytochem 45:375-82, 1997; Luehrsen et al., J Histochem Cytochem 48:133-45, 2000). Although up to 30 labels can be incorporated by these methods, detection sensitivity is variable, and not solely dependent on the number of labels incorporated. Labeling during synthesis can produce probes with labels on both or either end of the probe or internal to the probe sequence. Internal labels can be added to the ODN, but this method has been shown to reduce the stability of the probe/target complex, making it a less desirable method *(see* Brakel, *supra*). Although enzymatic tailing adds more labels per probe molecule, the length of the tail varies by experiment, leading to inconsistent results.

One recent approach for labeling deoxynucleotides, developed by Kreatech (Amsterdam, The Netherlands), involves use of platinum complex reagents to label probes *(see* van Belkum et al., Biotechniques 16:148-53, 1994; Wiegant et al., Cytogenet Cell Genet 87:47-52, 1999; Heetebrij et al., Chembiochem 4:573-83, 2003). This technology,

termed "ULS," is compatible with a variety of reporter molecules and is widely sub-licensed along with platinum coordination reagents. Although ULS has been used to label synthetic oligonucleotides FISH probes, due to poor labeling efficiency and the limitation that labels can only be attached to sequences internal to the ODN, this approach is not widely used.

In the current study, repeat sequences were targeted using ODN having 30 nucleotides of a chromosome-specific repeat sequence and 28mer G-tail. All human centromeres were detected, confirming adequate hybridization signal to visualize highly repeated DNA sequences in the human genome. 6 Y chromosome G-tail ODN probes were previously designed and hybridized to normal human metaphases. For the ODN design, only the absence of classical satellite 3 pentamer, TTCCA, and the GC% in the probe were taken into account. Following these criteria, 6 Y specific probes containing a 28mer G-Tail were designed and tested. Although 2 probes were not specific for the Y heterochromatin, four probes were specific. The signal from G-tail labeled ODN probes was more than two times higher than the background. These results underscore the importance of optimum probe design, supported by publicly available bioinformatics tools.

*4. G-tail ULS labeling of ODNs*

In the present study, a novel approach for ULS-labeling of ODN probes is used. Labeled probes, targeting specific repeated motifs localized to the human X and Y chromosomes, are generated by ULS labeling of synthetic ODNs having a long G-rich tail. By adding a long G-rich 5' tail in our probe design, the number of guanine nucleotides that can function as platinum acceptor molecules for ULS labeling is significantly increased. Efficiency of the ULS system has been reported as the addition of 1 label for every 10 available guanine nucleotides. *(See* Wiegant et al., Cytogenet Cell Genet 87:47-52, 1999; van Gijlswijk et al., Expert Rev Mol Diagn 1:81-91, 2001; van de Rijke et al., Anal Biochem 321:71-8, 2003.) By engineering G-tail ODN probes to contain over 20 guanines as part of the tail, statistically, up to 2 labels can be included in the probe. While stochastically internal guanines can serve as acceptor nucleotides for ULS, the G-content of the tail is 3 times higher than the number found in a typical probe sequence.

ULS G-tail labeled ODN probes have significant advantages compared with current commercial probes. For example, ODN probes can be synthesized, purified, and labeled in two days compared with conventional BAC probes which require at least nine days to produce a finished product. Further, it is estimated that the cost of production for ULS G-tail labeled ODN probes, including the cost of licensing, is 35-50% lower than the cost of conventional probes.

<u>B. Materials and Methods</u>

*1. ODNprobes specific for repeat sequences*

Chromosome specific probes were selected as described in Example 1 above and synthesized to include a 28-base G-Tail consisting of 8 copies of a (gggh) repeat, where h denotes A, C or T nucleotide. The G-Tail oligonucleotide design is shown below:

5'-ggghggghggghggghggghggghggghgggh(specific sequence)-3' (H= A, C or T)

To increase signal intensity of our probes, the optimal length of the G-tail is determined. The most sensitive and specific probe is synthesized to contain either 40mer or 52mer G-tail, containing 30 and 39 Gs, respectively. These longer tails permit incorporation of additional fluorescent labels in the ODN probe. The probe with the 28mer is also synthesized with the G-tail at both ends. Longer tails on both ends of the ODN probe can also be synthesized.

*2. G-Tail Oligoprobe Labeling*

G-tail probes are labeled using the Universal Labeling System (ULS, Kreatech), following manufacturer's instructions. To maximize labeling of the G-tail ODN, different ratios of labeling reagent to ODN probe mass are evaluated. As defined by manufacturer's protocols, standard labeling conditions consist of 1 unit of ULS reagent to label 1 μg of nucleic acid *(see* van Gijlswijk et al., Expert Rev Mol Diagn 1:81-91, 2001). 0.5, 1 and 3 units of labeling reagent per μg of ODN are evaluated and the optimal labeling using FISH analysis is determined.

*3. Conventional Probes*

To assess performance of G-Tail labeled probes, FISH results are compared to those obtained using commercially available FISH probes (Vysis/Abbott).

*4. Cytogenetic Slides*

Human chromosome slides are obtained by harvesting peripheral blood cultures of 5 chromosomally normal males. 0.5 to one mL peripheral blood per 25 mL culture flask from each individual is cultured in 10 mL RPMI 1640, 2 mM L-glutamine, FBS 10%, 250 μL PHA at 37°C and 5% $CO_2$. After 72 hours of culture, the cells are blocked in mitosis by adding 6 μl of colcemid (Karyomax, Invitrogen) per ml of culture. After 20 min at 37°C, the cultures are centrifuged 10 min at 1750 rpm, the supernatant is discarded, and 10 mL of hypotonic solution, 75 mM KCl, is carefully added. After incubating for 20 min at 37°C, several drops of Carnoy's fixative (Methanol:acetic acid 3:1) are added to the tubes in order to perform a prefixation of the cells and facilitate further fixation without cell clumping. After centrifugation, the cells are resuspended in Carnoy's fixative. The fixation step is repeated 3 times until the pellets are clearly white. During the last fixation, the correct amount of fixative required for slide preparation is evaluated. Spreading is done at ~22°C and ~50% humidity on SuperFrost slides with one or two drops of cell suspension per slide. The slides are kept overnight at 37°C and then stored at -20°C in hermetic boxes with a desiccant, until FISH is performed.

*5.* In Situ *Hybridization*

Cellular DNA is denatured by incubating in 70% formamide/2XSSC for 2 min at 72°C. Slides are then immediately placed in cold 70% ethanol for 2 min and dehydration is continued in 80%, 90%, and 100% ethanol, for 2min each and then air dried. 10 μL probe solution are placed on each spot or spread and covered with a Parafilm™ coverslip. Hybridization is done at 37°C using several time points from 1 min to 17 hours (overnight). Probe solution contains 20-50% formamide, 2XSSC, 10% Dextran Sulfate as well as 0.02-0.1mM labeled probe and 1μg of sonicated salmon sperm DNA. For each probe, optimal probe and formamide concentration is determined empirically. After hybridization, slides are washed at 45°C in 20-50% formamide/2XSSC for 3 min 3 times, followed by 5 washes with 2XSSC for 2 min, and one wash with 1XPhosphate Buffer with Detergent (1XPBD: 100 mM $Na_2HPO_4$, 6 mM $NaH_2PO_4$, 0.025% Nonidet P40 at pH 8). Finally, the slides are kept in a 1XPBD bath at room temperature. Probes are visualized using epifluorescence microscopy after mounting and DNA counterstaining with antifade solution containing DAPI 200 ng/mL.

*6. Fluorescence Microscopy and Image Acquisition*

Fluorescence microscopy analysis and digital image capture is performed using a Nikon Eclipse E600 microscope (Nikon Instruments, Melville, NY) equipped with a cooled CCD camera (photometric Ltd., Tucson, AZ). Images are captured using IP Lab Spectrum software (Scanalytics, Inc., Fairfax, VA), and final images are merged using Photoshop™ software (Adobe Systems Inc., San Jose, CA).

*7. Use of G-tail labeled probes on cells from human embryos*

The use of the G-tail labeled probes on human embryos is performed according to the materials and procedures described above. Chromosome-specific DNA repeat motifs are identified for at least 6 additional human chromosomes that can be targeted using G-tail labeled ODN probes (chr. Y). The use of these probes are validated in multicolor hybridization studies for PGD.

<u>Example 5: Preparation of multicolor probe cocktail</u>

A multicolor probe kit is prepared by mixing equal amounts of chromosome 2, 4, and Y chromosome ODN probe cocktails. Chromosome 2, 4, and Y chromosome probe cocktails for inclusion in the multicolor probe kit are selected based on relative specificity of various chromosome 2, 4, and Y chromosome probes, as well as the optimum number of ODN probe(s) for each cocktail, as determined in hybridization assays such as described above. Each different probe is labeled with a different fluorophore. For example a Chromosome 2 ODN probe is labeled with Alexa 488 (excitation maximum at 492 nm and emission maximum at 520 nm); a Chromosome 4 ODN probe is labeled with Alexa 546 (excitation maximum at 555 nm and emission maximum at 570 nm); and a Y Chromosome ODN probe is labeled with Alexa 594 (excitation maximum at 588 nm and emission maximum at 615 nm.

Using the multicolor chromosome 2, 4, and Y chromosome probe cocktail, the minimum hybridization time is determined by hybridizing the multicolor cocktail on 5 chromosomally normal samples at different times: from 1 min to 2 hours. For these studies, signal intensity on 30 interphase nuclei, per individual and tested hybridization time, are calculated. Optimum hybridization time is the shortest time that results in a signal to noise ratio ≥ commercial probes.

Example 6: Use of G-tailed labeled oligonucleotide probes in detection of precursor ribosomal RNA in the cell nucleolus

An oligonucleotide complementary to a region of the nascent rRNA was synthesized to contain a G-tail, then ULS labeled using Alexa-568 or Alexa 488 reagents. *In situ* hybridization using the Alexa 568 labeled probe and the Alexa 488 labeled probe both resulted in specific detection of nascent ribosomal RNA. Hybridization signal was restricted to nucleoli. Signal patterns were indistinguishable from non-G-tail labeled control ODN probes.

Example 7: Use of G-tailed labeled oligonucleotide probes for detection of mRNA

Sixty G-tailed ODN probes (labeled with biotin by the ULS method) were used for detection of Ki-67 mRNA in SUPT-1 cells. Ki-67 mRNA was reliably detected, even in not fully optimized *in situ* hybridization experiments.

Sixty G-tailed ODN probes, specific for Ki-67 mRNA, were labeled with biotin by the ULS method. SUPT-1 cells were fixed with saponin-containing paraformaldehyde and hybridized 16 hours with the G-tailed biotin-labeled ODN probes, either with only labeled ODN probes or with 100-fold excess of non-labeled probes added to labeled probes. After hybridization, tyramide signal amplification was performed using AlexaFluor 546 tyramide and other reagents from Molecular Probes (Eugene, OR) TSA kit. Ki-67 mRNA was reliably detected, even in not fully optimized *in situ* hybridization experiments. Fluorescence of single cells measured by CCD camera was calculated with the help of IPLab (Fairfax, VA) imaging software. Mean values of cells in A and B was 1014 and 314, respectively. Signal/noise for this measurements was, therefore, 1014/314=3.22.

Example 8: Use of 5' end labeled oligonucleotide probes for detection of bladder cancer

Following the technique described by Meiers et al. in 2007 for the preparation of slides of urothelial cells, FISH can be performed in record time on these cells to detect aneuploidy using the oligo-FISH probes (Meiers et al., "Improved filter method for urine sediment detection of urothelial carcinoma by fluorescence in situ hybridization," Arch Pathol Lab Med. 131(10):1574-7 (2007)). Using oligonucleotides instead of genomic DNA probes cloned in plasmids or BACs allows detection of urothelial carcinoma in just 2 hours.

*1. Slide preparation*

Urine samples were preserved with ethanol and drawn up into a 60mL syringe threaded with a Luer lock tip. An 8μ filter mounted in a 13mm filter holder (Millipore, Billerica, MA) was attached to the syringe tip. The urine sample was then pushed gently through the filter until filter saturation. The membrane was then placed on a positively charged glass slide and gentlyly pushed with a Kimwipe to transfer the epithelial cells on the glass surface. Slides were immediately fixed in freshly made methanol:acetic acid 3:1 for 5 minutes. The slides were air dryed and aged for 15min at 65°C.

*2. Slides pre-treatments*

In order to improve probe access to cellular DNA, slides were treated in a solution of RNAse for 30min. followed by a 15min treatment in protease. Finally the slides were fixed in 1% formaldehyde for 10min.

*3. Cellular DNA denaturation*

Prior to the application of the probes, cellular DNA was denatured by incubating the slides for 5min in 70% Formamide in 2xSSC at 72°C. Then the slides were dehydrated in cold Ethanol.

*4. Oligo-FISHprobe hybridization*

A combination of oligo-FISH probes for detection of chromosomes 3, 7, 9 and 17 was applied on the slide and incubated for 5min. at 37°C. Slides were then collected in 2xSSC at room temperature until coverslips easily separated from the slides. Slides were then washed for 2min in a bath of 0.2xSSC 0.1% SDS at 50°C under agitation. Slides were collected again in a bath of 2xSSC at room temperature and mounted with antifade solution containing DAPI as a counterstain.

*5. Slide analysis and interpretation*

Slides were observed under an epifluorescence microscope equipped with the filters corresponding to the probe fluors. An aneuploid cell was defined as a cell showing a number of signals for a particular chromosome different

than 2. If a minimum of 4 cells were observed with the same aneuploidy, the sample was considered positive for urothelial carcinoma.

It is understood that the examples and embodiments described herein are for illustrative purposes only and that various modifications or changes in light thereof will be suggested to persons skilled in the art and are to be included within the spirit and purview of this application and scope of the appended claims. All publications, Genbank Accession Nos., patents, and patent applications cited herein are hereby incorporated by reference in their entirety for all purposes. The invention is further described with reference to the following numbered paragraphs:

1. A synthetic oligonucleotide comprising:

   a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes.

2. The oligonucleotide of paragraph 1, wherein the repeat sequence is a member selected from the group consisting of a centromeric repeat, a pericentromeric repeat, a heterochromatin repeat, and a telomeric repeat.
3. The oligonucleotide of paragraph 1, wherein the consensus repeat sequence is selected from the group consisting of an alpha satellite, beta satellite, gamma satellite, satellite 1, satellite 2 and a satellite 3 repeat.
4. The oligonucleotide of paragraph 1, wherein the chromosome-specific sequence is less than 84% identical to all other contiguous nucleic acid sequences within the human genome.
5. The oligonucleotide of paragraph 1, wherein the chromosome-specific sequence is specific for an alpha monomer repeat sequence from a chromosome selected from the group consisting of the Y chromosome, chromosome 2, and chromosome 4.
6. The oligonucleotide of paragraph 5, wherein the chromosome-specific sequence is selected from the group consisting of:

| | |
|---|---|
| CCAGTCGAATCCATTCGAGTACATACC | (SEQ ID NO:1); |
| CCTTTTGAATCCATTCCATTGGAGTCC | (SEQ ID NO:2); |
| ATTCATTGCATTCCGTTTCATGAAATTCGA | (SEQ ID NO:3); |
| CTGCATACAATTTCACTCCATTCGTTCCCA | (SEQ ID NO:4); |
| TCCATTGGAGTCAATTCCTTTCGACACCCA | (SEQ ID NO:5); |
| TTGATCCTATTTTATTAAATTGCATTCTAT | (SEQ ID NO:6); |
| GTGCGCCCTCAACTAACAGTGTTGAAGCTT | (SEQ ID NO:7); |
| GAAACGGGATTGTCTTCATATAAACTCTAG | (SEQ ID NO:8); |
| GTATCTTCCAATAAAAGCTAGATAGAAGCA | (SEQ ID NO:9); |
| ATGTCAGAAACTTTTTCATGATGTATCTAC | (SEQ ID NO:10); |
| TATGTGTGATGTGCGCCCTCAACTAAGAGT | (SEQ ID NO:11); |
| TCTCAGAAGCTTCATTGGGATGTTTCAATT | (SEQ ID NO:12); |
| GGAATACGGTGATAAAGGAAATATCTTCCA | (SEQ ID NO:13); |
| TCTTTGTGTTGTGTGTACTCATGTAACAGT | (SEQ ID NO:14); |
| TTTCTGCCCTACCTGGAAGCGGACATTTCG | (SEQ ID NO:15); |
| GGTTATCTTCATATAAAATCCAGACAGGAG | (SEQ ID NO:16); |
| CGGCACTACCTGGAAGTGGATATTTCGAGC | (SEQ ID NO:17); |
| TCTGCACTACCTGGAAGAGGCCATTTCGAG | (SEQ ID NO:18); and |
| CCTACGGGGAGAAAGGAAATATCTTCAAAT | (SEQ ID NO:19). |

7. The oligonucleotide of paragraph 1, further comprising a detectable label.
8. The oligonucleotide of paragraph 7, wherein the detectable label comprises a fluorophore.
9. The oligonucleotide of paragraph 7, wherein the fluorophore is selected from the group consisting of fluorescein, rhodamine, Texas Red, phycoerythrin, Cy3, Cy5, Alexa 532, Alexa 546, Alexa 568, and Alexa 594.
10. The oligonucleotide of paragraph 1, further comprising an oligonucleotide that forms a hairpin structure comprising a stem region and a loop region, wherein a plurality of nucleotides within the hairpin structure have an attached detectable label.
11. The oligonucleotide of paragraph 10, wherein the stem region has 16-40 nucleotides.
12. The oligonucleotide of paragraph 10, wherein at least two nucleotides having an attached detectable label are adjacent.

13. The oligonucleotide of paragraph 10, wherein the nucleotides having an attached detectable label are spaced at least two nucleotides apart.

14. The oligonucleotide of paragraph 10, wherein the nucleotides having an attached detectable label are spaced three nucleotides apart.

15. A composition comprising a plurality of oligonucleotides according to paragraph 7.

16. The composition of paragraph 15, wherein the detectable label for each of the oligonucleotides is a fluorophore having a spectrally distinguishable emission wavelength.

17. A labeled probe comprising:

a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes; and
a detectable label.

18. The probe of paragraph 17, wherein the detectable label is a direct label.

19. The probe of paragraph 18, wherein the direct label comprises a fluorophore.

20. The probe of paragraph 19, wherein the fluorophore is selected from the group consisting of fluorescein, rhodamine, Texas Red, phycoerythrin, Cy3, Cy5, Alexa 532, Alexa 546, Alexa 568, and Alexa 594.

21. The probe of paragraph 17, wherein the label is an indirect label.

22. The probe of paragraph 21, wherein the indirect label comprises a fluorophore.

23. The probe of paragraph 22, wherein the fluorophore is selected from the group consisting of fluorescein, rhodamine, Texas Red, phycoerythrin, Cy3, Cy5, Alexa 532, Alexa 546, Alexa 568, and Alexa 594.

24. The probe of paragraph 17, further comprising an oligonucleotide that forms a hairpin structure comprising a stem region and a loop region, wherein a plurality of nucleotides within the hairpin structure have an attached detectable label.

25. The probe of paragraph 24, wherein the stem region has 16-40 nucleotides.

26. The probe of paragraph 24, wherein at least two nucleotides having an attached detectable label are adjacent.

27. The probe of paragraph 24, wherein the nucleotides having an attached detectable label are spaced at least two nucleotides apart.

28. The probe of paragraph 24, wherein the nucleotides having an attached detectable label are spaced three nucleotides apart.

29. A probe cocktail composition comprising:

a plurality of different labeled probes, each different labeled probe comprising a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes and having

(a) a different chromosome-specific oligonucleotide; and
(b) a different detectable label that is distinguishable from each of the other detectable labels.

30. The composition of paragraph 29, wherein each detectable label is a fluorophore having a spectrally distinguishable emission wavelength.

31. The composition of paragraph 29, wherein each probe further comprises an oligonucleotide that forms a hairpin structure comprising a stem region and a loop region, wherein a plurality of nucleotides within the hairpin structure have an attached detectable label.

32. A method of identifying a chromosome-specific probe sequence, the method comprising:

(a) comparing a repeat sequence of a specific human chromosome with a consensus repeat sequence of all human chromosomes; and
(b) identifying a nucleic acid sequence consisting of 25-35 contiguous nucleotides within the repeat sequence of the specific human chromosome and having less than 84% identity to the consensus repeat sequence as a potential chromosome-specific probe sequence.

33. The method of paragraph 32, further comprising

(c) comparing the chromosome specific probe sequence identified in (b) with all other contiguous nucleotide sequences within the human genome; and
(d) identifying a nucleic acid sequence that has more than 16% mismatch to all other contiguous nucleotide sequences

within the human genome as a potential chromosome specific probe sequence.

34. The method of paragraph 33, further comprising

(e) chemically synthesizing and labeling the chromosome specific probe sequence with a detectable label;
(f) performing *in situ* hybridization on a member selected from the group consisting of human metaphases and human interphases using the labeled probe sequence of (e);
(g) detecting the signal associated with the label, wherein a chromosome specific probe sequence that produces a signal at least 2 fold higher than the background upon hybridization is selected as a chromosome specific probe.

35. The method of paragraph 32, wherein the repeat sequence is selected from the group consisting of a centromeric repeat sequence, a pericentromeric repeat sequence, a heterochromatin repeat sequence, and a telomeric repeat sequence.
36. The method of paragraph 32, wherein the repeat sequence is selected from the group consisting of an alpha satellite, beta satellite, gamma satellite, satellite 1, satellite 2 and a satellite 3 repeat.
37. The method of paragraph 32, wherein the consensus repeat sequence is selected from the group consisting of a centromeric repeat sequence, a pericentromeric repeat sequence, a heterochromatin repeat sequence, and a telomeric repeat sequence.
38. The method of paragraph 32, wherein the consensus repeat sequence is selected from the group consisting of an alpha satellite, beta satellite, gamma satellite, satellite 1, satellite 2 and a satellite 3 repeat.
39. The method of paragraph 32, wherein the specific human chromosome is selected from the group consisting of the Y chromosome, chromosome 2, and chromosome 4.
40. A method of detecting a specific human chromosome, the method comprising:

(a) contacting a preparation of human chromosomes with a labeled oligonucleotide comprising a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes;
(b) incubating the chromosome preparation and the oligonucleotide under conditions sufficient to allow the oligonucleotide to hybridize to, if present, a specific human chromosome having a repeat sequence perfectly complementary to the chromosome-specific sequence of the probe; and
(c) detecting the label to detect the specific human chromosome, if present.

41. The method of paragraph 40, wherein the label comprises a fluorophore.
42. The method of paragraph 41, wherein the fluorophore is selected from the group consisting offluorescein, rhodamine, Texas Red, phycoerythrin, Cy3, Cy5, Alexa 532, Alexa 546, Alexa 568, and Alexa 594.
43. The method of paragraph 40, wherein the label is attached to the hairpin portion of an oligonucleotide that forms a hairpin structure comprising a stem region and a loop region.
44. The method of paragraph 43, wherein a plurality of the nucleotides in the hairpin structure have an attached label.
45. The method of paragraph 43, wherein the stem region has 16-40 nucleotides.
46. The method of paragraph 43, wherein at least two nucleotides having an attached label are adjacent.
47. The method of paragraph 43, wherein the nucleotides having an attached label are spaced at least two nucleotides apart.
48. The method of paragraph 43, wherein the nucleotides having an attached detectable label are spaced three nucleotides apart.
49. The method of paragraph 40, wherein detection of the human chromosome identifies a chromosomal abnormality.
50. The method of paragraph 40, wherein the chromosome preparation comprises a human cell and the hybridization step is performed *in situ* on chromosomes within the cell.
51. A method of detecting a specific human chromosome, the method comprising:

(a) contacting a preparation of human chromosomes with a probe cocktail composition comprising a plurality of different labeled oligonucleotides having a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes, wherein each different oligonucleotide has a different chromosome- specific sequence and a different detectable label that is distinguishable from each of the other detectable labels;
(b) incubating the chromosome preparation and the probe cocktail composition under conditions sufficient to allow the oligonucleotides to hybridize to, if present, a specific human chromosome having a repeat sequence perfectly

complementary to the chromosome-specific sequence of the oligonucleotide; and
(c) detecting the label(s) to detect the specific human chromosome(s), if present.

52. A method of selecting a chromosome-specific probe sequence, the method comprising:

(a) comparing centromeric repeat sequences of a specific human chromosome with a consensus alpha-monomer repeat sequence of centromeric repeat sequences of all human chromosomes; and
(b) identifying a nucleic acid sequence consisting of 25-35 contiguous nucleotides within the specific centromeric repeat sequence and having less than 84% identity to the consensus alpha-monomer repeat sequence, thereby selecting a chromosome-specific probe sequence.

53. The method of paragraph 52, further comprising preparing an oligonucleotide probe comprising a chromosome-specific nucleic acid sequence, said sequence consisting of the chromosome-specific sequence selected in (b).
54. The method of paragraph 52, which comprises identifying a plurality of nucleic acid sequences in (b), thereby selecting a plurality of chromosome-specific probe sequences.
55. The method of paragraph 54, further comprising
preparing a plurality of labeled oligonucleotide probes, each probe comprising a detectable label and a different chromosome-specific nucleic acid sequence, each different sequence being one of the chromosome-specific sequences selected in (b);
performing, individually with each of the different oligonucleotide probes, *in situ* hybridization on a member selected from the group consisting of human metaphases and human interphases;
detecting a signal associated with the label for each of the hybridized probes; and selecting one or more of the oligonucleotide probes based on the degree of detected signal.
56. The method of paragraph 54, further comprising

(c) comparing the nucleic acid sequences identified in (b) with all other contiguous nucleotide sequences within the human genome; and
(d) discarding any nucleic acid sequences that have more than 84% identity to another contiguous nucleotide sequence within the human genome.

57. The method of paragraph 33, which comprises identifying a plurality of nucleic acid sequences in (b) and (d), thereby identifying a plurality of potential chromosome-specific probe sequences.
58. The method of paragraph 57, further comprising
preparing a plurality of labeled oligonucleotide probes, each probe comprising a detectable label and a different chromosome-specific nucleic acid sequence, each different sequence being one of the chromosome-specific sequences identified in (d);
performing, individually with each of the different oligonucleotide probes, *in situ* hybridization on a member selected from the group consisting of human metaphases and human interphases;
detecting a signal associated with the label for each of the hybridized probes; and
selecting one or more of the oligonucleotide probes based on the degree of detected signal.
59. A method for preparing a labeled probe, the method comprising:

attaching an oligonucleotide that forms a hairpin structure comprising a stem region and a loop region, wherein a plurality of nucleotides within the hairpin structure have an attached detectable label, to a chromosome-specific nucleic acid sequence consisting of 25-35 contiguous nucleotides perfectly complementary to a centromeric repeat sequence on a specific human chromosome, wherein the chromosome-specific sequence is less than 84% identical to a consensus alpha-monomer repeat sequence of centromeric repeat sequences of all human chromosomes.

60. The method of paragraph 59, wherein the stem region has 16-40 nucleotides.
61. The method of paragraph 59, wherein at least two nucleotides having an attached detectable label are adjacent.
62. The method of paragraph 59, wherein the nucleotides having an attached detectable label are spaced at least two nucleotides apart.
63. The method of paragraph 59, wherein the nucleotides having an attached detectable label are spaced three nucleotides apart.
64. A method of detecting a specific human chromosome, the method comprising:

contacting a preparation of human chromosomes with a labeled probe comprising a chromosome-specific sequence consisting of 25-35 contiguous nucleotides perfectly complementary to a repeat sequence on a specific human

chromosome, wherein the chromosome-specific sequence is less than 84% identical to a consensus repeat sequence of all human chromosomes;
incubating the chromosome preparation and the probe under conditions sufficient to allow the probe to hybridize to, if present, a specific human chromosome having a centromeric repeat sequence perfectly complementary to the chromosome-specific sequence of the probe; and
detecting the label to detect the specific human chromosome, if present.

65. The method of paragraph 64, which comprises contacting the preparation of human chromosomes with a plurality of different labeled probes, each different labeled probe having a different chromosome-specific sequence and a different detectable label that is distinguishable from each of the other detectable labels.
66. The method of paragraph 65, wherein each detectable label is a fiuorophore having a spectrally distinguishable emission wavelength.
67. The method of paragraph 64, which is a method for identifying a chromosomal abnormality.
68. The method of paragraph 64, wherein detecting the label comprises microscopic imaging.
69. The method of paragraph 64, wherein detecting the label comprises image scanning microscopy.
70. The method of paragraph 64, wherein the chromosome preparation comprises a human cell and the hybridization step is performed *in situ* on chromosomes within the cell.
71. A kit for detection of a specific human chromosome in a cell, the kit comprising:

a labeled oligonucleotide comprising a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes.

72. The kit of paragraph 71, wherein the label is attached to the chromosome- specific sequence via a linker.
73. The kit of paragraph 71, wherein the labeled oligonucleotide further comprises a sequence that forms a hairpin structure comprising a stem region and a loop region, wherein a plurality of nucleotides within the hairpin structure have an attached detectable label.
74. The kit of paragraph 73, wherein the stem region has 16-40 nucleotides.
75. The kit of paragraph 73, wherein at least two nucleotides having an attached detectable label are adjacent.
76. The kit of paragraph 73, wherein the nucleotides having an attached detectable label are spaced at least two nucleotides apart.
77. The kit of paragraph 73, wherein the nucleotides having an attached detectable label are spaced three nucleotides apart.
78. A kit for detection of a specific human chromosome in a cell, the kit comprising:

a plurality of different labeled probes, each different labeled probe comprising a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes and having

(a) a different chromosome-specific sequence; and
(b) a different detectable label that is distinguishable from each of the other detectable labels.

79. The kit of paragraph 78, wherein each detectable label is a fluorophore having a spectrally distinguishable emission wavelength.

## Claims

**1.** A method of detecting a specific human chromosome, the method comprising:

(a) contacting a preparation of human chromosomes with a labeled oligonucleotide comprising a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes;
(b) incubating the chromosome preparation and the oligonucleotide under conditions sufficient to allow the oligonucleotide to hybridize to, if present, a specific human chromosome having a repeat sequence perfectly complementary to the chromosome-specific sequence of the probe; and
(c) detecting the label to detect the specific human chromosome, if present.

**2.** The method of claim 1, wherein the label comprises a fluorophore.

**3.** The method of claim 2, wherein the fluorophore is selected from the group consisting of fluorescein, rhodamine, Texas Red, phycoerythrin, Cy3, Cy5, Alexa 532, Alexa 546, Alexa 568, and Alexa 594.

**4.** The method of claim 1, wherein the label is attached to the hairpin portion of an oligonucleotide that forms a hairpin structure comprising a stem region and a loop region.

**5.** The method of claim 4, wherein a plurality of the nucleotides in the hairpin structure have an attached label.

**6.** The method of claim 4, wherein the stem region has 16-40 nucleotides.

**7.** The method of claim 4, wherein at least two nucleotides having an attached label are adjacent.

**8.** The method of claim 4, wherein the nucleotides having an attached label are spaced at least two nucleotides apart.

**9.** The method of claim 4, wherein the nucleotides having an attached detectable label are spaced three nucleotides apart.

**10.** The method of claim 1, wherein detection of the human chromosome identifies a chromosomal abnormality.

**11.** The method of claim 1, wherein the chromosome preparation comprises a human cell and the hybridization step is performed in situ on chromosomes within the cell.

**12.** A method of detecting a specific human chromosome, the method comprising:

(a) contacting a preparation of human chromosomes with a probe cocktail composition comprising a plurality of different labeled oligonucleotides having a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes, wherein each different oligonucleotide has a different chromosome-specific sequence and a different detectable label that is distinguishable from each of the other detectable labels;
(b) incubating the chromosome preparation and the probe cocktail composition under conditions sufficient to allow the oligonucleotides to hybridize to, if present, a specific human chromosome having a repeat sequence perfectly complementary to the chromosome-specific sequence of the oligonucleotide; and
(c) detecting the label(s) to detect the specific human chromosome(s), if present.

**13.** A method of detecting a specific human chromosome, the method comprising:

contacting a preparation of human chromosomes with a labeled probe comprising a chromosome-specific sequence consisting of 25-35 contiguous nucleotides perfectly complementary to a repeat sequence on a specific human chromosome, wherein the chromosome-specific sequence is less than 84% identical to a consensus repeat sequence of all human chromosomes;
incubating the chromosome preparation and the probe under conditions sufficient to allow the probe to hybridize to, if present, a specific human chromosome having a centromeric repeat sequence perfectly complementary to the chromosome-specific sequence of the probe; and
detecting the label to detect the specific human chromosome, if present.

**14.** The method of claim 13, which comprises contacting the preparation of human chromosomes with a plurality of

different labeled probes, each different labeled probe having a different chromosome-specific sequence and a different detectable label that is distinguishable from each of the other detectable labels.

**15.** The method of claim 14, wherein each detectable label is a fluorophore having a spectrally distinguishable emission wavelength.

**16.** The method of claim 14, which is a method for identifying a chromosomal abnormality.

**17.** The method of claim 14, wherein detecting the label comprises microscopic imaging.

**18.** The method of claim 14, wherein detecting the label comprises image scanning microscopy.

**19.** The method of claim 14, wherein the chromosome preparation comprises a human cell and the hybridization step is performed in situ on chromosomes within the cell.

Figure 1

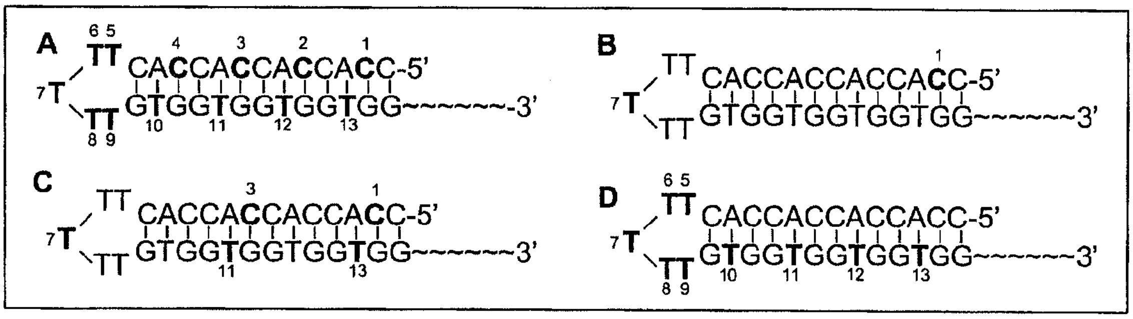
A
CACCACCACCACC-5'
GTGGTGGTGGTGG~~~~~~~-3'
B
CACCACCACCACC-5'
GTGGTGGTGGTGG~~~~~~3'
C
CACCACCACCACC-5'
GTGGTGGTGGTGG~~~~~~3'
D
CACCACCACCACC-5'
GTGGTGGTGGTGG~~~~~~3'

Europäisches Patentamt
European Patent Office
Office européen des brevets

# EUROPEAN SEARCH REPORT

Application Number
EP 15 16 7287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 96/00234 A1 (APROGENEX INC [US]) 4 January 1996 (1996-01-04)<br>* page 13, paragraph 3 *<br>* page 33, paragraph 1 - paragraph 2 *<br>* page 39, paragraph 1 *<br>* page 41, paragraph 1 *<br>* claims 32-35; examples 1-3 *<br>----- | 1-3, 10-19 | INV.<br>C07H21/00<br>C12Q1/68 |
| X | MATERA A G ET AL: "Oligonucleotide probes for the analysis of specific repetitive DNA sequences by fluorescence in situ hybridization.", HUMAN MOLECULAR GENETICS OCT 1992 LNKD-PUBMED:1307254, vol. 1, no. 7, October 1992 (1992-10), pages 535-539, XP009153413, ISSN: 0964-6906 | 1-3, 10-19 | |
| Y | * the whole document *<br>----- | 4-9 | |
| X | A. GREGORY MATERA ET AL: "An oligonucleotide probe specific to the centromeric region of human chromosome 5", GENOMICS, vol. 18, no. 3, December 1993 (1993-12), pages 729-731, XP055010228, ISSN: 0888-7543, DOI: 10.1016/S0888-7543(05)80387-8<br>* the whole document *<br>----- | 1-3, 10-19 | TECHNICAL FIELDS SEARCHED (IPC)<br>C12Q |
| | -/-- | | |

The present search report has been drawn up for all claims

| Place of search | Date of completion of the search | Examiner |
|---|---|---|
| Munich | 28 July 2015 | Eveleigh, Anna |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 16 7287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | C. O'KEEFE ET AL: "Oligonucleotide probes for alpha satellite DNA variants can distinguish homologous chromosomes by FISH", HUMAN MOLECULAR GENETICS, vol. 5, no. 11, November 1996 (1996-11), pages 1793-1799, XP055204573, ISSN: 0964-6906, DOI: 10.1093/hmg/5.11.1793 * the whole document * ----- | 1-3, 10-19 | |
| X | TANEJA K L ET AL: "Multicolor fluorescence in situ hybridization with peptide nucleic acid probes for enumeration of specific chromosomes in human cells", GENES, CHROMOSOMES & CANCER, XX, XX, vol. 30, no. 1, 2001, pages 57-63, XP002324770, DOI: 10.1002/1098-2264(2000)9999:9999<::AID-GCC1054>3.0.CO;2-M * the whole document * ----- | 1-3, 10-19 | TECHNICAL FIELDS SEARCHED (IPC) |
| Y | WO 2005/007815 A2 (ONE CELL SYSTEMS INC [US]; MOEN JR PHILLIP T [US]; TRNOVSKY JAN [US]) 27 January 2005 (2005-01-27) * paragraphs [0007], [0023], [0052], [0071], [0079], [0100]; figures 2,4,6-7; example 1 * ----- | 4-9 | |
| | -/-- | | |

The present search report has been drawn up for all claims

3

| Place of search | Date of completion of the search | Examiner |
|---|---|---|
| Munich | 28 July 2015 | Eveleigh, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 16 7287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE EMBL [Online]<br><br>16 July 1988 (1988-07-16), "Human alpha-satellite consensus sequence", XP002662003, retrieved from EBI accession no. EM_GSS:X07685 Database accession no. X07685<br>* the whole document *<br>----- | 1-19 | |
| A | HENEGARIU ET AL: "Small marker chromosome identification in metaphase and interphase using centromeric multiplex fish (CM-FISH).", LABORATORY INVESTIGATION, vol. 81, no. 4, April 2001 (2001-04), pages 475-481, XP055010322, ISSN: 0023-6837<br>* the whole document *<br>----- | 1-19 | |
| | | | TECHNICAL FIELDS SEARCHED (IPC) |
| A | YUROV Y B ET AL: "High resolution multicolor fluorescence in situ hybridization using cyanine and fluorescein dyes: rapid chromosome identification by directly fluorescently labeled alphoid DNA probes.", HUMAN GENETICS MAR 1996, vol. 97, no. 3, March 1996 (1996-03), pages 390-398, XP009185554, ISSN: 0340-6717<br>* the whole document *<br>----- | 1-19 | |
| A | US 5 064 948 A (MOYZIS ROBERT K [US] ET AL) 12 November 1991 (1991-11-12)<br>* the whole document *<br>-----<br>-/-- | 1-19 | |

The present search report has been drawn up for all claims

3

| Place of search | Date of completion of the search | Examiner |
|---|---|---|
| Munich | 28 July 2015 | Eveleigh, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 16 7287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MATERA A G ET AL: "A PERINUCLEOLAR COMPARTMENT CONTAINS SEVERAL RNA POLYMERASE III TRANSCRIPTS AS WELL AS THE POLYPYRIMIDINE TRACT-BINDING PROTEIN,hnRNP I", THE JOURNAL OF CELL BIOLOGY : JCB, THE ROCKEFELLER UNIVERSITY PRESS, US, vol. 129, no. 5, June 1995 (1995-06), pages 1181-1193, XP003010743, ISSN: 0021-9525, DOI: 10.1083/JCB.129.5.1181 * page 1183, left-hand column, paragraph 2 * ----- | 17,18 | |

TECHNICAL FIELDS SEARCHED (IPC)

The present search report has been drawn up for all claims

3

| Place of search | Date of completion of the search | Examiner |
|---|---|---|
| Munich | 28 July 2015 | Eveleigh, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 16 7287

This annex lists the patent family members relating to the patent documents cited in the above-mentioned European search report. The members are as contained in the European Patent Office EDP file on
The European Patent Office is in no way liable for these particulars which are merely given for the purpose of information.

28-07-2015

| Patent document cited in search report | Publication date | Patent family member(s) | Publication date |
|---|---|---|---|
| WO 9600234 A1 | 04-01-1996 | AU 2871695 A | 19-01-1996 |
| | | WO 9600234 A1 | 04-01-1996 |
| WO 2005007815 A2 | 27-01-2005 | AU 2004257200 A1 | 27-01-2005 |
| | | CA 2531560 A1 | 27-01-2005 |
| | | CN 1836050 A | 20-09-2006 |
| | | EP 1668157 A2 | 14-06-2006 |
| | | JP 2007529994 A | 01-11-2007 |
| | | NZ 544959 A | 28-02-2009 |
| | | US 2005059049 A1 | 17-03-2005 |
| | | WO 2005007815 A2 | 27-01-2005 |
| US 5064948 A | 12-11-1991 | AU 2986889 A | 17-08-1989 |
| | | CA 1332921 C | 08-11-1994 |
| | | DE 3904270 A1 | 24-08-1989 |
| | | FR 2627192 A1 | 18-08-1989 |
| | | GB 2215724 A | 27-09-1989 |
| | | JP 2777170 B2 | 16-07-1998 |
| | | JP H02215400 A | 28-08-1990 |
| | | US 5064948 A | 12-11-1991 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 96225807 P
- US 6300066 B
- WO 9201699 A
- US 5714327 A
- US 6825330 B
- WO 9635696 A
- WO 9815564 A
- US 5082830 A
- US 5580990 A
- WO 9815546 A
- US 5719024 A


### Non-patent literature cited in the description


- **HEIM ; MITELMAN.** *Cancer Cytogenetics, Chromosomal and Molecular Genetic Aberrations of Tumor Cells,* 1995
- **KLINGER.** *Cytogenetics Analysis, Non-Isotopic Methods in Molecular Biology, A Practical Approach,* 1995
- **TIMM et al.** *Cytometry,* 1995, vol. 22, 250-5
- **HESELMEYER et al.** *Genes, Chromosomes & Cancer,* 1997, vol. 19, 233-40
- **SAUER et al.** *Apmis,* 2003, vol. 111, 444-50
- **JOBANPUTRA et al.** *Hum. Reprod,* 2002, vol. 17, 1166-70
- **MUNNÉ et al.** *Reprod Biomed Online,* 2003, vol. 7, 91-7
- **MARQUEZ et al.** *Reprod Biomed Online,* 2000, vol. 1, 729-31
- **ABDELHADI et al.** *Reprod Biomed Online,* 2003, vol. 6, 226-31
- **GIANAROLI et al.** *Fertil Steril,* 1999, vol. 72, 837-44
- **VON HANSEMANN D.** Ueber asymmetrische Zelltheilung in epithelkrebsen und deren biologische bedeutung. *Virchow's Arch Pathol Anat,* vol. 119, 299-326
- **DUESBERG et al.** Genetic instability of cancer cells is proportional to their degree of aneuploidy. *Proc Natl Acad Sci USA,* 1998, vol. 95, 13692-13697
- **DUESBERG et al.** Aneuploidy the somatic mutation that makes cancer a species on its own. *Cell Motil. Cytoskeleton,* 2000, vol. 47, 81-107
- **BIALY H.** Aneuploidy and cancer: vintage wine in a new bottle?. *Nat Biotechnol.,* 1998, vol. 16 (2), 137-8
- **SEN S.** Aneuploidy and cancer. *Curr Opin Oncol.,* 2000, vol. 12 (1), 82-8
- **SOKOLOVA et al.** *J Mol Diagn,* 2000, vol. 2 (3), 116-23
- **MATERA et al.** *Genomics,* 1993, vol. 18, 729-31
- **PELLESTOR et al.** *Cytogenet. Cell Genet,* 1995, vol. 70, 138-142
- **ANSTON et al.** *Eur. J. Hum. Genet.,* 2003, vol. 11, 357-363
- **PAULASOVA et al.** *Mol. Hum. Reprod.,* 2004, vol. 10, 467-472
- **O'KEEFE AND MATERA.** *Genome Res.,* 2000, vol. 10, 1342-1350
- **ALLSHIRE et al.** *Nucleic Acids Res,* 1989, vol. 17 (12), 4611-27
- **CHO et al.** *Nucleic Acids Res,* 1991, vol. 19 (6), 1179-82
- **FOWLER et al.** *Nucleic Acids Res,* 1987, vol. 15 (9), 3929
- **HAAF et al.** *Cell,* 1992, vol. 70 (4), 681-96
- **LEE et al.** *Chromosoma,* 2000, vol. 109 (6), 381-9
- **MAEDA ; SMITHIES.** *Annu Rev Genet,* 1986, vol. 20, 81-108
- **MEYNE ; GOODWIN.** *Chromosoma,* 1994, vol. 103 (2), 99-103
- Localized highly repetitive DNA sequences in vertebrate genomes. **MIKLOS.** Molecular evolutionary genetics. Plenum Publishing Corp, 1985, 241-321
- **TAGARRO et al.** *Hum Genet,* 1994, vol. 93 (2), 125-8
- **WAYE ; WILLARD.** *PNAS USA,* 1989, vol. 86 (16), 6250-4
- **WILLARD ; WAYE.** *J Mol Evol,* 1987, vol. 25 (3), 207-14
- **VISSEL ; CHOO.** *Nucleic Acids Res.,* 1987, vol. 15 (16), 6751-6752
- **NIELSEN et al.** *Science,* 1991, vol. 254, 1497-1500

- **NIELSEN et al.** *J. Biomol. Struct. Dyn,* vol. 17, 175-91
- **VARANI.** *Annu. Rev. Biophys. Biomol. Struct.,* 1995, vol. 24, 379-404
- **BRUCHEZ et al.** *Science,* 1998, vol. 81, 2013-2016
- **WARREN ; NIE.** *Science,* 1998, vol. 281, 2016-2018
- **HERNANDEZ-SANTOS et al.** *Anal. Chem.,* 2005, vol. 77, 2868-2874
- **GLICK ; PASTERNAK.** Molecular Biotechnology: Principles and Applications of Recombinant DNA. ASM Press, 1998
- **HERNANDEZ-SANTOSET et al.** *Anal. Chem.,* 2005, vol. 77, 2868-2874
- **RAAP et al.** *BioTechniques,* 2004, vol. 37, 1-6
- **HEETEBRIJ et al.** *ChemBioChem,* 2003, vol. 4, 573-583
- **VAN DE RIJKE et al.** *Analytical Biochemistry,* 2003, vol. 321, 71-78
- **GUPTA et al.** *Nucleic Acids Research,* 2003, vol. 31, e13
- **VAN GIJLSWIJK et al.** *Clinical Chemistry,* 2002, vol. 48, 1352-1359
- **ALERS et al.** *Genes, Chromosomes & Cancer,* 1999, vol. 25, 301-305
- **WIEGANT et al.** *Cytogenetics and Cell Genetics,* 1999, vol. 87, 7-52
- **JELSMA et al.** *Journal ofNIHResearch,* 1994, vol. 5, 82
- **VAN BELKUM et al.** *BioTechniques,* 1994, vol. 16, 148-153
- **VAN BELKUM et al.** *Journal of Virological Methods,* 1993, vol. 45, 189-200
- **SAMBROOK ; RUSSELL.** Molecular Cloning: A Laboratory Manual. Cold Spring Harbor Laboratory Press, 2001
- **AUSUBEL et al.** Short Protocols in Molecular Biology. John Wiley & Sons, 1999
- **HERMAN.** Bioconjuate Chemistry. Academic Press
- **WIEGANT et al.** *Cytogenet. Cell Genet,* 1999, vol. 87, 47-52
- **SMITH ; WATERMAN.** *Adv. Appl. Math.,* 1970, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **AUSUBEL et al.** *Current Protocols in Molecular Biology,* 1995
- **FENG ; DOOLITTLE.** *J. Mol. Evol,* 1987, vol. 35, 351-360
- **HIGGINS ; SHARP.** *CABIOS,* 1989, vol. 5, 151-153
- **FENG ; DOOLITTLE.** *J. Mol. Evol.,* 1987, vol. 35, 351-360
- **ALTSCHUL et al.** *Nuc. Acids Res.,* 1977, vol. 25, 3389-402
- **ALTSCHUL et al.** *J. Mol. Biol.,* 1990, vol. 215, 403-10
- **TRASK et al.** *Science,* 1985, vol. 230, 1401-1402
- Laboratory Techniques in Biochemistry and Molecular Biology. **TIJSSEN.** Hybridization with Nucleic Acid Probes. Elsevier, 1993, vol. 24
- **SCHROECK et al.** *Science,* 1996, vol. 273, 494-497
- **SPEICHER et al.** *Nature Genetics,* 1996, vol. 12, 368-375
- **GALL ; PARDUE.** *Methods in Enzymology,* 1981, vol. 21, 470-480
- **HENDERSON.** *International Review of Cytology,* 1982, vol. 76, 1-46
- **ANGERER et al.** Genetic Engineering: Principles and Methods. Plenum Press, 1985
- **MEHE et al.** *Cytometry,* 2000, vol. 42, 357-362
- **NAKAGOME et al.** *Cytogenet Cell Genet,* 1991, vol. 56, 74-7
- **FOWLER et al.** *Nucleic Acids Res,* 1987, vol. 15, 3929
- **NAKAHORI et al.** *Nucleic Acids Res,* 1986, vol. 14, 7569-80
- **WEIER et al.** *J Histochem Cytochem,* 1990, vol. 38, 421-6
- **TATUSOVA et al.** *FEMS Microbiol Lett,* 1999, vol. 174, 247-50
- **WAYE et al.** *Proc Natl Acad Sci USA,* 1989, vol. 86, 6250-4
- **MASHKOVA et al.** *Gene,* 1994, vol. 140 (2), 211-217
- **HAAF ; WILLARD.** *Genomics,* 1992, vol. 13, 122-128
- **ROCCHI et al.** *Genomics,* 1990, vol. 8, 705-709
- **MUNNE et al.** *Reprod Biomed Online,* 2003, vol. 7, 91-7
- **PINKEL et al.** *Proc Natl Acad Sci USA,* 1988, vol. 85, 9138-42
- **BRAKEL.** *End Labeled Nucleotide Probe,* 1992
- **VAN GIJLSWIJK et al.** *JHistochem Cytochem,* 1997, vol. 45, 375-82
- **LUEHRSEN et al.** *J Histochem Cytochem,* 2000, vol. 48, 133-45
- **VAN BELKUM et al.** *Biotechniques,* 1994, vol. 16, 148-53
- **WIEGANT et al.** *Cytogenet Cell Genet,* 1999, vol. 87, 47-52
- **HEETEBRIJ et al.** *Chembiochem,* 2003, vol. 4, 573-83
- **VAN GIJLSWIJK et al.** *Expert Rev Mol Diagn,* 2001, vol. 1, 81-91
- **VAN DE RIJKE et al.** *Anal Biochem,* 2003, vol. 321, 71-8

• **MEIERS et al.** Improved filter method for urine sediment detection of urothelial carcinoma by fluorescence in situ hybridization. *Arch Pathol Lab Med.,* 2007, vol. 131 (10), 1574-7

SEQUENCE LISTING

```
<110>  Cellay, LLC
       Aurich-Costa, Joan
       Bradley, Sean P.
       Moen, Phillip T.

<120>  HIGHLY VISIBLE CHROMOSOME-SPECIFIC PROBES AND RELATED METHODS

<130>  019168-000910PC

<150>  US 60/962,258
<151>  2007-07-26

<160>  19

<170>  PatentIn version 3.4

<210>  1
<211>  27
<212>  DNA
<213>  Artificial

<220>
<223>  Synthetic Y1

<400>  1
ccagtcgaat ccattcgagt acatacc                                          27


<210>  2
<211>  27
<212>  DNA
<213>  Artificial

<220>
<223>  Synthetic Y2

<400>  2
cctttgaat ccattccatt ggagtcc                                           27


<210>  3
<211>  30
<212>  DNA
<213>  Artificial

<220>
<223>  Synthetic Y3

<400>  3
attcattgca ttccgtttca tgaaattcga                                       30


<210>  4
<211>  30
<212>  DNA
<213>  Artificial

<220>
<223>  Synthetic Y4

<400>  4
```

ctgcatacaa tttcactcca ttcgttccca 30

<210> 5
<211> 30
<212> DNA
<213> Artificial

<220>
<223> Synthetic Y5

<400> 5
tccattggag tcaattcctt tcgacaccca 30

<210> 6
<211> 30
<212> DNA
<213> Artificial

<220>
<223> Synthetic Y6

<400> 6
ttgatcctat tttattaaat tgcattctat 30

<210> 7
<211> 30
<212> DNA
<213> Artificial

<220>
<223> Synthetic 2.1.1

<400> 7
gtgcgccctc aactaacagt gttgaagctt 30

<210> 8
<211> 30
<212> DNA
<213> Artificial

<220>
<223> Synthetic 2.2.2

<400> 8
gaaacgggat tgtcttcata taaactctag 30

<210> 9
<211> 30
<212> DNA
<213> Artificial

<220>
<223> Synthetic 2.5.1

<400> 9
gtatcttcca ataaaagcta gatagaagca 30

<210> 10
<211> 30
<212> DNA
<213> Artificial

<220>
<223> Synthetic 2.6.1

<400> 10
atgtcagaaa ctttttcatg atgtatctac 30

<210> 11
<211> 30
<212> DNA
<213> Artificial

<220>
<223> Synthetic 2.7.3

<400> 11
tatgtgtgat gtgcgccctc aactaagagt 30

<210> 12
<211> 30
<212> DNA
<213> Artificial

<220>
<223> Synthetic 2.8.4

<400> 12
tctcagaagc ttcattggga tgtttcaatt 30

<210> 13
<211> 30
<212> DNA
<213> Artificial

<220>
<223> Synthetic 2.10.1

<400> 13
ggaatacggt gataaaggaa atatcttcca 30

<210> 14
<211> 30
<212> DNA
<213> Artificial

<220>
<223> Synthetic 4.3.2

<400> 14
tctttgtgtt gtgtgtactc atgtaacagt 30

<210> 15
<211> 30
<212> DNA

```
<213>  Artificial

<220>
<223>  Synthetic 4.6.2

<400>  15
tttctgccct acctggaagc ggacatttcg                                      30


<210>  16
<211>  30
<212>  DNA
<213>  Artificial

<220>
<223>  Synthetic 4.7.5

<400>  16
ggttatcttc atataaaatc cagacaggag                                      30


<210>  17
<211>  30
<212>  DNA
<213>  Artificial

<220>
<223>  Synthetic 4.10.2

<400>  17
cggcactacc tggaagtgga tatttcgagc                                      30


<210>  18
<211>  30
<212>  DNA
<213>  Artificial

<220>
<223>  Synthetic 4.18.7

<400>  18
tctgcactac ctggaagagg ccatttcgag                                      30


<210>  19
<211>  30
<212>  DNA
<213>  Artificial

<220>
<223>  Synthetic 4.22.10

<400>  19
cctacgggga gaaaggaaat atcttcaaat                                      30
```